(19) Europäisches Patentamt
European Patent Office
Office européen des brevets

(11) Veröffentlichungsnummer: **0 116 889**
**B1**

(12) # EUROPÄISCHE PATENTSCHRIFT

(45) Veröffentlichungstag der Patentschrift: **23.04.86**

(51) Int. Cl.⁴: **C 07 C 43/172**, A 01 N 31/04

(21) Anmeldenummer: **84101207.3**

(22) Anmeldetag: **07.02.84**

(54) **Cyclopropylmethyl(en)ether, Verfahren zu ihrer Herstellung und ihre Verwendung in Schädlingsbekämpfungsmitteln.**

(30) Priorität: **19.02.83 DE 3305835**

(43) Veröffentlichungstag der Anmeldung: **29.08.84 Patentblatt 84/35**

(45) Bekanntmachung des Hinweises auf die Patenterteilung: **23.04.86 Patentblatt 86/17**

(84) Benannte Vertragsstaaten: **AT BE CH DE FR GB IT LI NL**

(56) Entgegenhaltungen:
**EP - A - 0 005 710**
**US - A - 4 008 287**

**CHEMICAL ABSTRACTS, Band 88, Nr. 23, 5. Juni 1978, Seite 540, Nr. 169560k, Columbus, Ohio, USA I.G. TISHCHENKO et al.: "Synthesis of ortho esters from vinylacetylenic ethers"**
**"Chemie der Pflanzenschutz- und Schädlingsbekämpfungsmittel", Band 7, K. Naumann: "Chemie der synthetischen Pyrethroid-Insektizide", 1981, Seiten 3-6, Springer-Verlag, Berlin, DE.**

(73) Patentinhaber: **BAYER AG, Konzernverwaltung RP Patentabteilung, D-5090 Leverkusen 1 Bayerwerk (DE)**

(72) Erfinder: **Krüger, Bernd-Wieland, Dr., Sillerstrasse 49, D-5600 Wuppertal 1 (DE)**
Erfinder: **Priesnitz, Uwe, Dr., Severinstrasse 58, D-5650 Solingen 1 (DE)**
Erfinder: **Jäger, Gerhard, Dr., Gellertstrasse 18, D-5090 Leverkusen 1 (DE)**
Erfinder: **Behrenz, Wolfgang, Dr., Untergründemich 14, D-5063 Overath (DE)**

**EP 0 116 889 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte Cyclopropylmethyl(en)ether, mehrere Verfahren zu ihrer Herstellung sowie ihre Verwendung in Schädlingsbekämpfungsmitteln, vorzugsweise zur Bekämpfung von Arthropoden, insbesondere von Insekten, Milben und Spinnentieren.

Synergistische Mischungen von insektiziden Wirkstoffen, z.B. von Pyrethroiden mit bestimmten Methylendioxyphenyl-Derivaten, z.B. Piperonylbutoxid als Synergisten sind bereits bekannt geworden (vgl. z.B. K. Naumann, „Chemie der Pflanzenschutz- und Schädlingsbekämpfungsmittel", Springer-Verlag Berlin, Band 7 (1981), Seiten 3 bis 6). In der Praxis ist die Wirksamkeit solcher Präparate jedoch nicht immer voll befriedigend.

Es wurden die neuen substituierten Cyclopropylmethyl(en)ether der allgemeinen Formel (I)

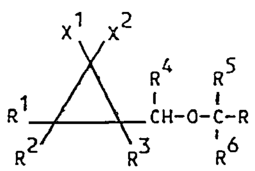

(I)

in welcher

$X^1$ und $X^2$ gleich oder verschieden sind und für Fluor, Chlor und/oder Brom stehen,

$R^1$ für Wasserstoff oder $C_1$-$C_6$-Alkyl steht,

$R^2$ und $R^3$ gleich oder verschieden sind und für Wasserstoff, $C_1$-$C_6$-Alkyl, $C_1$-$C_4$-Halogenalkyl, 1-Pyrazolylmethyl, 1-Imidazolylmethyl, 1-(1,2,4-Triazolyl)methyl, $C_1$-$C_4$-Alkoxy-$C_1$-$C_4$-Alkyl, $C_1$-$C_4$-Alkylthio-$C_1$-$C_4$-Alkyl, (Di)$C_1$-$C_4$-Alkylamino-$C_1$-$C_4$-Alkyl oder für gegebenenfalls durch Halogen, wie insbesondere Chlor, Brom oder Iod, substituierte Reste der Reihe Phenyl, $C_2$-$C_4$-Alkenoxy-$C_1$-$C_2$-Alkyl und $C_2$-$C_4$-Alkinoxy-$C_1$-$C_2$-Alkyl stehen,

$R^4$ für Wasserstoff oder $C_1$-$C_4$-Alkyl steht,

$R^5$ und $R^6$ gleich oder verschieden sind und für Wasserstoff, $C_1$-$C_4$-Alkyl oder für gegebenenfalls durch Halogen, wie insbesondere Chlor, Brom oder Iod substituierte Reste der Reihe Phenyl, Benzyl und Phenylethyl stehen, und

R für Triiod-$C_2$-$C_4$-alkenyl, Dibromiod-$C_2$-$C_4$-alkenyl, Dichloriod-$C_2$-$C_4$-alkenyl, $C_2$-$C_4$-Alkinyl oder Iod-$C_2$-$C_4$-alkinyl steht, wobei sich die Mehrfachbindungen jeweils am Ende der Kette befinden und im Falle von Trihalogenalkenyl ein Iodatom an das letzte Kohlenstoffatom und die beiden übrigen Halogenatome an die beiden letzten Kohlenstoffatome der Kette gebunden sind und im Falle von Iodalkinyl das Iodatom an das letzte Kohlenstoffatom der Kette gebunden ist,

gefunden, die als Synergisten in Schädlingsbekämpfungsmitteln verwendet werden können, welche zusätzlich gegen Arthropoden, vorzugsweise gegen Insekten und Spinnentiere, insbesondere gegen Insekten wirksame Stoffe enthalten.

Als gegen Arthropoden wirksame Stoffe kommen praktisch alle üblichen Wirkstoffe in Frage (vgl. z.B. K.H. Büchel, „Pflanzenschutz- und Schädlingsbekämpfungsmittel", Thieme-Verlag Stuttgart, 1977, und „Farm Chemicals Handbook", 1979, Meister Publishing Co, Willougby, 1979).

Weiterhin wurde gefunden, dass man die neuen substituierten Cyclopropylmethyl(en)ether der Formel (I) erhält, wenn man

a) Cyclopropylmethyl(en)halogenide der allgemeinen Formel (II)

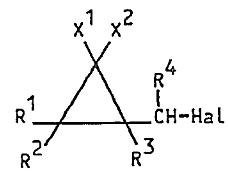

(II)

in welcher

$R^1$, $R^2$, $R^3$, $R^4$, $X^1$ und $X^2$ die oben angegebene Bedeutung haben, und Hal für Halogen, wie insbesondere Chlor, Brom oder Iod steht,

mit Alkoholen der allgemeinen Formel (III)

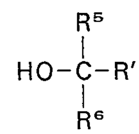

(III)

in welcher

$R^5$ und $R^6$ die oben angegebene Bedeutung haben, und

R' für $C_2$-$C_4$-Alkinyl steht, wobei sich die Mehrfachbindung am Ende der Kette befindet,

oder deren Salze, gegebenenfalls in Gegenwart von Säureakzeptoren und/oder gegebenenfalls in Gegenwart von Verdünnungsmitteln, umsetzt, oder indem man

b) die nach Verfahrensvariante (a) hergestellten neuen Verbindungen der allgemeinen Formel (Ia)

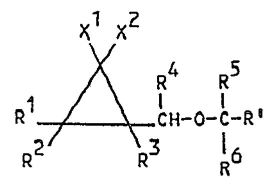

(Ia)

in welcher $R^1$, $R^2$, $R^3$, $R^4$, $R^5$, $R^6$, R', $X^1$ und $X^2$ die oben angegebenen Bedeutungen haben,

mit 0,5 bis 6 Mol Iod in Gegenwart von Säureakzeptoren und gegebenenfalls in Gegenwart eines Verdünnungsmittels umsetzt, oder indem man

c) die nach Verfahren (b) hergestellten neuen Verbindungen der allgemeinen Formel (Ib)

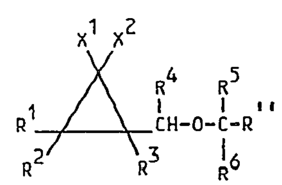

(Ib)

in welcher $R^1$, $R^2$, $R^3$, $R^4$, $R^5$, $R^6$, $X^1$ und $X^2$ die oben angegebenen Bedeutungen haben, und

R'' für Iod-$C_2$-$C_4$-Alkinyl steht, wobei sich die Mehrfachbindung am Ende der Kette befindet und

das Iodatom an das letzte Kohlenstoffatom der Kette gebunden ist,
mit Chlor, Brom oder Iod gegebenenfalls in Gegenwart eines Verdünnungsmittels umsetzt.

Die Alkylreste $R^1$, $R^2$, $R^3$, $R^4$, $R^5$ und $R^6$, die wie in den Patentansprüchen angegeben gegebenenfalls substituiert sein können, sind geradkettig oder verzweigt. Beispielhaft seien gegebenenfalls substituiertes Methyl, Ethyl, n- und iso-Propyl, n-, iso-, sek.- und tert.-Butyl genannt.

Als Iodalkinyl R bzw. R″ und Alkinyl R bzw. R′ seien beispielsweise Ethinyl, Propinyl-(2) und Butinyl-(3) und die entsprechenden, in den Patentansprüchen näher definierten Iod-Derivate genannt.

Halogenalkyl $R^2$ und $R^3$ enthalten im Alkylteil 1 bis 4 Kohlenstoffatome, insbesondere 1 bis 2 Kohlenstoffatome und vorzugsweise 1 bis 5, insbesondere 1 bis 3 Halogenatome, wobei die Halogenatome gleich oder verschieden sind und als Halogenatome, vorzugsweise Chlor oder Brom, stehen. Beispielhaft seien Chlormethyl oder Brommethylgenannt.

Alkoxyalkyl und Alkylthioalkyl $R^2$ und $R^3$ enthalten je Alkylteil 1 bis 4 Kohlenstoffatome, insbesondere 1 bis 2 Kohlenstoffatome, wobei der Alkylteil geradkettig oder verzweigt sein kann. Beispielhaft seien Methoxymethyl, Ethoxymethyl, Methoxyethyl, Ethoxyethyl, Methylthiomethyl, Methylthioethyl, Ethylthiomethyl und Ethylthioethyl genannt.

Als gegebenenfalls substituiertes Alkenoxyalkyl $R^2$ und $R^3$ steht gegebenenfalls im Alkenoxyteil und/oder Alkylteil substituiertes Alkenoxyalkyl mit 2 bis 4 Kohlenstoffatomen im Alkenoxyteil und 1 oder 2 Kohlenstoffatomen im Alkylteil, wobei der Alkylteil geradkettig oder verzweigt sein kann. Beispielhaft seien gegebenenfalls substituiertes Propenyloxymethyl, Butenyloxymethyl und Methylpropenyloxymethyl genannt.

Als gegebenenfalls substituiertes Alkinoxyalkyl $R^2$ und $R^3$ steht gegebenenfalls im Alkinoxyteil und/oder Alkylteil substituiertes Alkinoxyalkyl mit 2 bis 4 Kohlenstoffatomen im Alkinoxyteil und 1 oder 2 Kohlenstoffatomen im Alkylteil, wobei der Alkylteil geradkettig oder verzweigt sein kann. Beispielhaft seien gegebenenfalls substituiertes Propinyloxymethyl, Butinyloxymethyl und Methylpropinyloxy genannt.

(Di)Alkylaminoalkyl $R^2$ und $R^3$ enthalten im Alkylteil 1 bis 4 Kohlenstoffatome, insbesondere 1 bis 2 Kohlenstoffatome. Beispielhaft seien (Di)Methylaminomethyl, (Di)Methylaminoethyl, (Di)-Ethylaminomethyl und (Di)Ethylaminoethyl genannt. (Di)Alkylaminoalkyl bedeutet Mono- oder Dialkylaminoalkyl.

Halogen (oder Halogenatom) bedeutet Fluor, Chlor, Brom und Iod, vorzugsweise (wo nichts anderes angegeben ist) Fluor, Chlor oder Brom und besonders bevorzugt Chlor oder Brom.

Die Erfindung betrifft insbesondere Verbindungen der Formel (I), in welcher

$X^1$ und $X^2$ gleich oder verschieden sind und für Chlor und/oder Brom stehen,

$R^1$ für Wasserstoff, Methyl, Ethyl, n-Propyl oder n-Butyl steht,

$R^2$ und $R^3$ gleich oder verschieden sind und für Wasserstoff, Methyl, Ethyl, n-Propyl, n-Butyl, 1-Pyrazolylmethyl, 1-Imidazolylmethyl, 1-(1,2,4-Triazolyl)-methyl, Methoxymethyl, Ethoxymethyl, Ethoxyethyl, Methylthiomethyl, Ethylthiomethyl, Ethylthioethyl, (Di)Methylaminomethyl, (Di)-Methylaminoethyl, (Di)Ethylaminomethyl, (Di)-Ethylaminoethyl, Propenyloxymethyl, Propinyloxymethyl, Iodpropinyloxymethyl, Triiodpropenyloxymethyl, Dichloriodpropenyloxymethyl, Dibromiodpropenyloxymethyl, Phenyl, 2-, 3-, 4-Chlorphenyl, 2,4-Dichlorphenyl, 2-, 3-, 4-Bromphenyl, 2,4-Dibromphenyl, Chlormethyl und Brommethyl stehen,

$R^4$ für Wasserstoff, Methyl, Ethyl, n- oder i-Propyl steht, $R^5$ und $R^6$ gleich oder verschieden sind und für Wasserstoff, Methyl, Ethyl, n-Propyl, i-Propyl, 2-, 3-, 4-Chlorphenyl, 2,4-Dichlorphenyl oder 4-Bromphenyl stehen, und

R für Triiodethenyl, Dichloriodethenyl, Dibromiodethenyl, Ethinyl, Iodethinyl, Propinyl oder Iodpropinyl steht, wobei in den Iod enthaltenden Restenein Iodatom jeweils an das letzte Kohlenstoffatom der Kette gebunden ist.

Ganz besonders bevorzugt sind Verbindungen der Formel (I),
in welcher

$X^1$ und $X^2$ gleich oder verschieden sind und für Chlor und/oder Brom stehen,

$R^1$ für Wasserstoff, Methyl oder Ethyl steht,

$R^2$ und $R^3$ gleich oder verschieden sind und für Wasserstoff, Methyl, Ethyl, Chlormethyl, Brommethyl, 1-Propenyloxymethyl, 1-Propinyloxymethyl oder 1-Iodpropinyloxymethyl stehen,

$R^4$ für Wasserstoff oder Methyl steht,

$R^5$ und $R^6$ gleich oder verschieden sind und für Wasserstoff oder Methyl stehen, und

R für Triiodethenyl, Dichloriodethenyl, Dibromiodethenyl, Ethinyl oder Iodethinyl steht, wobei in den Iod enthaltenden Resten ein Iodatom jeweils an das letzte Kohlenstoffatom der Kette gebunden ist.

Verwendet man für die Verfahrensvariante (a) als Ausgangsstoffe für die Herstellung der Verbindungen der Formel (Ia) 2,2-Dichlorcyclopropylmethylbromid und 1-Propin-3-ol, so kann die Reaktion durch folgendes Formelschema skizziert werden:

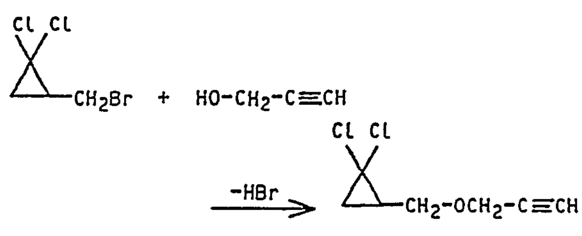


Verwendet man für die Verfahrensvariante (b) als Ausgangsstoffe für die Herstellung der Verbindungen der Formel (Ib) 3-(2,2-Dichlorcyclopropylmethoxy)-1-propin und Iod, so kann die Reaktion durch folgende Formelschemen skizziert werden:

(1) 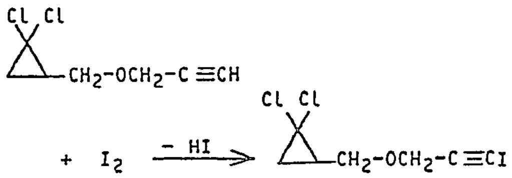


(2) 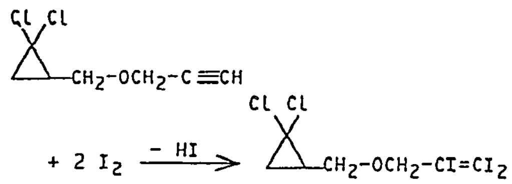


Verwendet man für die Verfahrensvariante (c) als Ausgangsstoffe 3-(2,2-Dichlorcyclopropylmethoxy)-1-iod-1-propin und Chlor, so kann die Reaktion durch folgendes Formelschema skizziert werden:

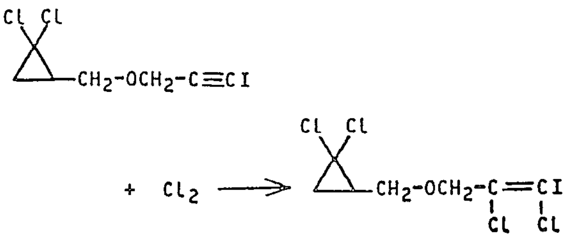


Die bei der erfindungsgemässen Verfahrensvariante (a) als Ausgangsstoffe zu verwendenden Cyclopropylmethyl(en)halogenide sind durch die Formel (II) definiert. In dieser Formel stehen $X^1$, $X^2$, $R^1$, $R^2$, $R^3$ und $R^4$ für diejenigen Reste, welche oben bei der Definition in Formel (I) angegeben sind. Hal steht in dieser Formel für Halogen, insbesondere für Chlor, Brom oder Iod.

Als Beispiele für die Verbindungen der Formel (II) seien genannt:

*Tabelle 1*

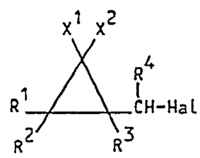 (II)

Hal = Chlor, Brom oder Iod

| $X^1$ | $X^2$ | $R^1$ | $R^2$ | $R^3$ | $R^4$ |
|---|---|---|---|---|---|
| Cl | Cl | H | $CH_3$ | H | H |
| Br | Br | H | $CH_3$ | H | H |
| Cl | Cl | H | H | H | H |
| Br | Br | H | H | H | H |
| Cl | Cl | H | H | $CH_3$ | H |
| Br | Br | H | H | $CH_3$ | H |
| Cl | Cl | H | $CH_3$ | $CH_3$ | H |
| Br | Br | H | $CH_3$ | $CH_3$ | H |
| Cl | Cl | H | $CH_3$ | $CH_3$ | $CH_3$ |
| Br | Br | H | $CH_3$ | $CH_3$ | $CH_3$ |
| Cl | Cl | H | H | H | $CH_3$ |
| Br | Br | H | H | H | $CH_3$ |
| Cl | Cl | H | $CH_3$ | H | $CH_3$ |
| Br | Br | H | $CH_3$ | H | $CH_3$ |
| Cl | Cl | H | H | $CH_3$ | $CH_3$ |
| Br | Br | H | H | $CH_3$ | $CH_3$ |
| Cl | Cl | H | $CH_2Cl$ | H | H |
| Br | Br | H | $CH_2Cl$ | H | H |
| Cl | Cl | H | $CH_2Br$ | H | H |
| Br | Br | H | $CH_2Br$ | H | H |
| Cl | Cl | H | H | $CH_2Cl$ | H |
| Br | Br | H | H | $CH_2Cl$ | H |
| Cl | Cl | H | H | $CH_2Br$ | H |
| Br | Br | H | H | $CH_2Br$ | H |
| Cl | Cl | H | $IC \equiv CH-CH_2OCH_2-$ | H | H |

| $X^1$ | $X^2$ | $R^1$ | $R^2$ | $R^3$ | $R^4$ |
|---|---|---|---|---|---|
| Br | Br | H | $IC{\equiv}CH{-}CH_2OCH_2{-}$ | H | H |
| Cl | Cl | H | $HC{\equiv}C{-}CH_2OCH_2{-}$ | H | H |
| Br | Br | H | $HC{\equiv}C{-}CH_2OCH_2{-}$ | H | H |
| Cl | Cl | H | 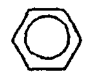 | H | H |
| Br | Br | H | 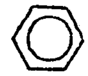 | H | H |
| Cl | Cl | $CH_3$ | $CH_3$ | H | H |
| Br | Br | $CH_3$ | $CH_3$ | H | H |
| Cl | Cl | $CH_3$ | $CH_3$ | H | $CH_3$ |
| Br | Br | $CH_3$ | $CH_3$ | H | $CH_3$ |

Die Verbindungen der Formel (II) sind bekannt und/oder können in üblicher Weise nach an sich bekannten Verfahren hergestellt werden (vgl. „Angew. Chem. Int. Ed.", *16*, 493 (1977) Verlag Chemie, Weinheim und „Phase Transfer Catalysis", 1980, Verlag Chemie, Weinheim).

Die weiter bei der Verfahrensvariante (a) als Ausgangsstoffe zu verwendenden Alkohole sind durch die Formel (III) definiert. In dieser Formel stehen $R^5$, $R^6$ und R' für diejenigen Reste, welche oben bei der Definition in Formel (I) bzw. (III) angegeben sind.

Als Salze der Verbindungen der Formel III kommen die (gegebenenfalls in situ vorliegenden) Alkali- oder Erdalkalisalze, wie Natrium, Kalium- oder Calciumsalze in Frage.

Als Beispiele für die Verbindungen der Formel (III) seien genannt:

*Tabelle 2*

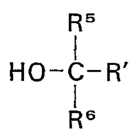 (III)

$HO{-}CH_2{-}C{\equiv}CH$

$HO{-}CH(CH_3){-}C{\equiv}CH$

$HO{-}C(CH_3)_2{-}C{\equiv}CH$

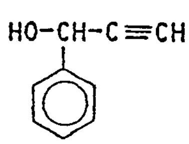

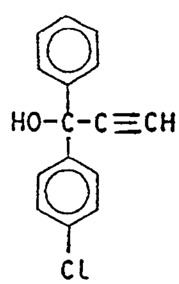

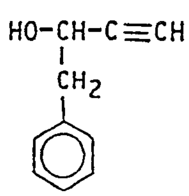

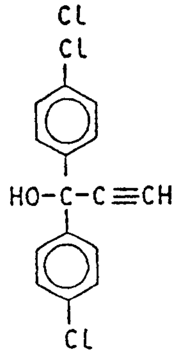

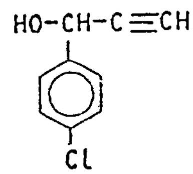

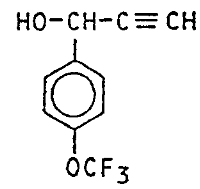

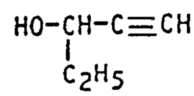

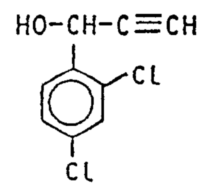

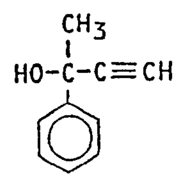

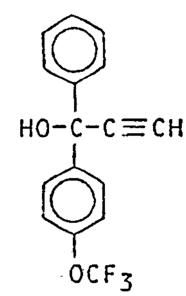

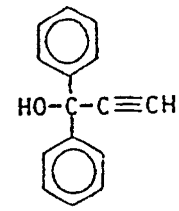

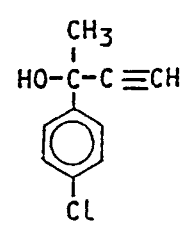

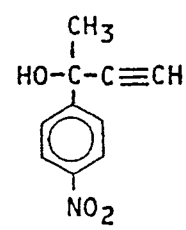

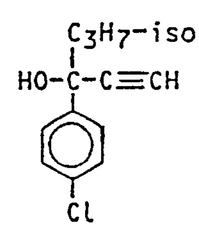

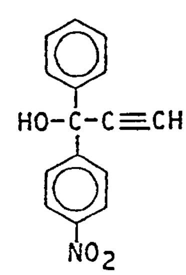

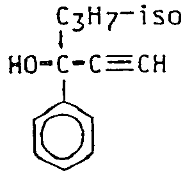

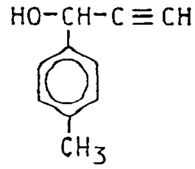

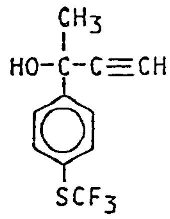

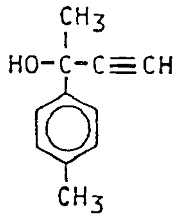

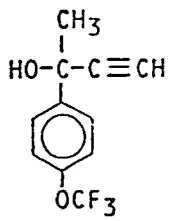

$HO-CH_2-CH_2-C\equiv CH$

$HO-CH(CH_3)-CH_2-C\equiv CH$

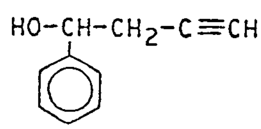

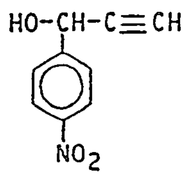

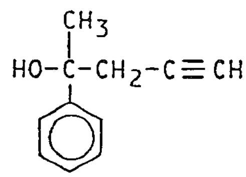

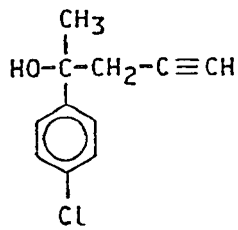

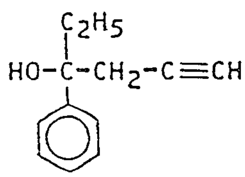

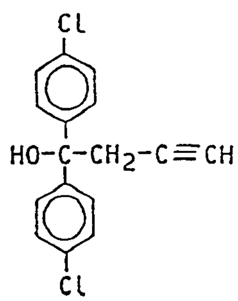

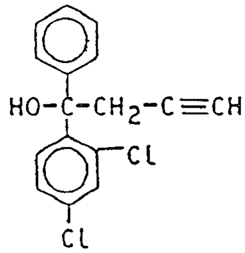

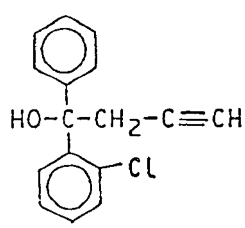

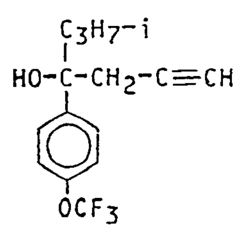

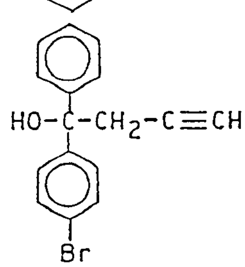

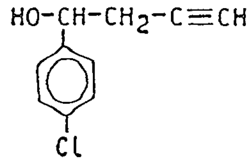

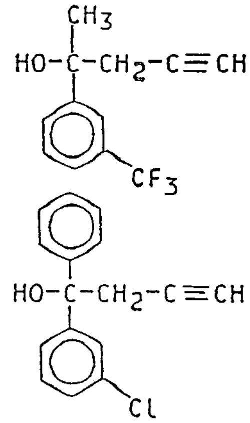

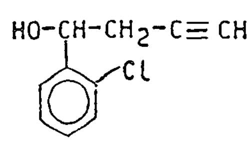

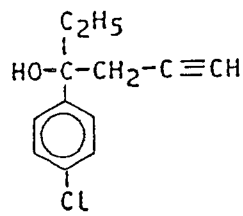

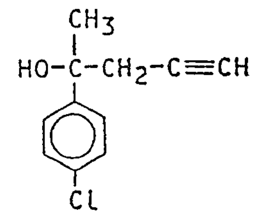

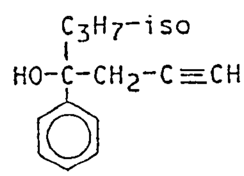

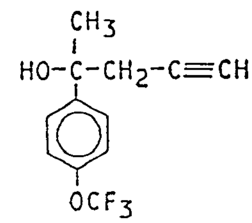

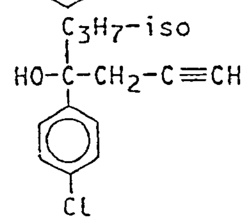

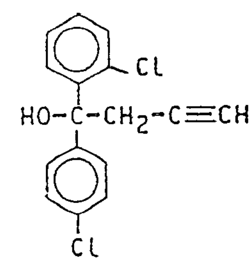

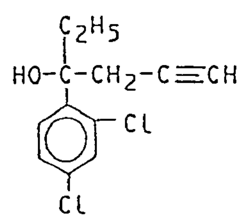

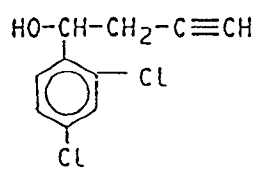

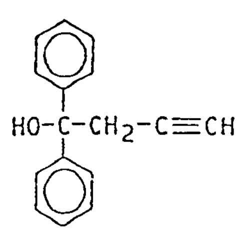

Die Alkohole der Formel (III) sind bekannt oder können in üblicher Weise nach an sich bekannten Verfahren hergestellt werden (vgl. DE-AS 1217368; „J. Agr. Food Chem.", *18*, 78 (1970); Liebigs Ann. Chem.", *308*, 264 (1899) und *682*, 62 (1965); „Angew. Chem.", *71*, 245 (1959); „J. Am. Chem. Soc." *83*, 4990 (1961); „Bull. Soc. Chim. France", 911 (1969) und DE-OS 3122176.

Als Verdünnungsmittel kommen bei der Verfahrensvariante (a) praktisch alle inerten organischen Lösungsmittel in Frage. Hierzu gehören insbesondere aliphatische und aromatische, gegebenenfalls halogenierte, Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether, wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, ausserdem Ester, wie Essigsäuremethylester und -ethylester, ferner Nitrile, wie z.B. Acetonitril und Propionitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können bei der Verfahrensvariante (a) alle üblichen Säurebindemittel verwendet werden. Hierzu gehören vorzugsweise Alkalicarbonate, -hybride, -hydroxide und -alkoholate, wie Natrium- und Kaliumcarbonat, Natriumhydrid, Natriumhydroxid, Natrium- und Kaliummethylat bzw. -ethylat, ferner aliphatische, aromatische oder heterocyclische Amine, beispielsweise Trimethylamin, Triethylamin, Dimethylanilin, Dimethylbenzylamin und Pyridin.

Die Reaktionstemperatur wird bei der erfindungsgemässen Verfahrensvariante (a) zwischen etwa −20 und +90°C, vorzugsweise zwischen 0 und +80°C, gehalten. Das Verfahren wird vorzugsweise bei Normaldruck durchgeführt.

Bei der Durchführung der erfindungsgemässen Verfahrensvariante (a) setzt man auf 1 Mol der Verbindung mit der Formel (II) 1 bis 3 Mol, vorzugsweise 1 bis 2 Mol der Verbindung mit der Formel (III) ein. Im allgemeinen wird das erfindungsgemässe Verfahren wie folgt ausgeführt:

Zum vorgelegten Alkohol der Formel (III) wird ein Säureakzeptor gegeben und die Verbindung der Formel (II) wird portionsweise hinzugefügt. Nach der Umsetzung wird auf übliche Art und Weise die Verbindung der Formel (Ia) isoliert und gereinigt.

Die bei der erfindungsgemässen Verfahrensvariante (b) als Ausgangsstoffe zu verwendenden Verbindungen sind durch die Formel (Ia) definiert. In dieser Formel stehen $X^1$, $X^2$, $R^1$, $R^2$, $R^3$, $R^4$, $R^5$ und $R^6$ für diejenigen Reste, welche oben bei der Definition der Formel (I) angegeben sind. R' steht in dieser Formel bevorzugt für $C_2$-$C_4$-Alkinyl.

Als Beispiele für die Verbindungen der Formel (Ia) seien genannt:

*(Tabelle auf den nächsten Seiten)*

Die Verbindungen der Formel (Ia) sind neu. Die Herstellung der Verbindungen mit der Formel (Ia) erfolgt gemäss Verfahrensvariante (a).

Als Verdünnungsmittel kommen für die Umsetzung gemäss Verfahrensvariante (b) vorzugsweise die Solvenzien in Frage, die bei der Verfahrensvariante (a) bereits genannt wurden.

Als Säureakzeptoren kommen für die Umsetzung gemäss Verfahrensvariante (b) vorzugsweise die Säureakzeptoren in Frage, die bei der Verfahrensvariante (a) bereits genannt wurden.

Die Reaktionstemperatur wird beim erfindungsgemässen Verfahren (b) zwischen 0 und 80°C, vorzugsweise zwischen 20 und 60°C, gehalten. Das Verfahren wird vorzugsweise bei Normaldruck durchgeführt.

Bei der Durchführung der Verfahrensvariante (b) setzt man auf 1 Mol der Verbindung der Formel (Ia) 0,5 bis 6 Mol, vorzugsweise 1 bis 4 Mol Iod ein. Die Aufarbeitung und Isolierung der neuen Triiodalkenyl- bzw. Iodalkinyl-Derivate der Formel (I) geschieht auf übliche Art und Weise.

Die bei der erfindungsgemässen Verfahrensvariante (c) als Ausgangsstoffe zu verwendenden Verbindungen sind durch die Formel (Ib) definiert. In dieser Formel stehen $X^1$, $X^2$, $R^1$, $R^2$, $R^3$, $R^4$, $R^5$ und $R^6$ für diejenigen Reste, welche oben bei der Definition der Formel (I) angegeben sind. R" steht in dieser Formel bevorzugt für $C_2$-$C_4$-Iodalkinyl.

Als Beispiele für die Verbindungen der Formel (Ib) seien beispielsweise die Iodalkinyl-Derivate der Verbindungen der Formel (Ia), aufgeführt in Tabelle 3, genannt.

Die Verbindungen der Formel (Ib) sind neu. Die Herstellung der Verbindungen mit der Formel (Ib) erfolgt gemäss Verfahrensvariante (b).

Als Verdünnungsmittel kommen für die Umsetzung gemäss Verfahrensvariante (c) vorzugsweise die Solvenzien in Frage, die bei der Verfahrensvariante (a) bereits genannt wurden.

Die Reaktionstemperatur wird beim erfindungsgemässen Verfahren (c) zwischen 0 und 60°C, vorzugsweise zwischen 20 und 40°C, gehalten. Das Verfahren wird vorzugsweise bei Normaldruck durchgeführt.

Bei der Durchführung der Verfahrensvariante (c) setzt man auf 1 Mol der Verbindung der Formel (Ib) 1 bis 3 Mol, vorzugsweise 1,1 bis 2,0 Mol Halogen ein. Die Aufarbeitung und Isolierung der Trihalogenalkenyl-Derivate der Formel (I) geschieht auf übliche Art und Weise.

Die neuen Verbindungen fallen teilweise in Form von Ölen an, die sich zum Teil nicht unzersetzt destillieren lassen, jedoch durch sogenanntes „Andestillieren", d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

*Tabelle 3*

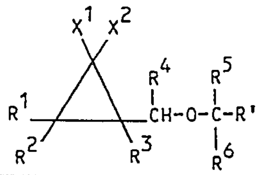

(Ia)

| X¹ | X² | R¹ | R² | R³ | R⁴ | $-CR^5R^6R'$ |
|---|---|---|---|---|---|---|
| Cl | Cl | H | $CH_3$ | H | H | $-CH_2-C{\equiv}CH$ |
| Br | Br | H | $CH_3$ | H | H | $-CH_2-C{\equiv}CH$ |
| Cl | Cl | H | H | H | H | $-CH_2-C{\equiv}CH$ |
| Br | Br | H | H | H | H | $-CH_2-C{\equiv}CH$ |
| Cl | Cl | H | H | $CH_3$ | H | $-CH_2-C{\equiv}CH$ |
| Br | Br | H | H | $CH_3$ | H | $-CH_2-C{\equiv}CH$ |
| Cl | Cl | H | $CH_3$ | $CH_3$ | H | $-CH_2-C{\equiv}CH$ |
| Br | Br | H | $CH_3$ | $CH_3$ | H | $-CH_2-C{\equiv}CH$ |
| Cl | Cl | H | $CH_3$ | $CH_3$ | $CH_3$ | $-CH_2-C{\equiv}CH$ |
| Br | Br | H | $CH_3$ | $CH_3$ | $CH_3$ | $-CH_2-C{\equiv}CH$ |
| Cl | Cl | H | H | H | $CH_3$ | $-CH_2-C{\equiv}CH$ |
| Br | Br | H | H | H | $CH_3$ | $-CH_2-C{\equiv}CH$ |
| Cl | Cl | H | $CH_3$ | H | $CH_3$ | $-CH_2-C{\equiv}CH$ |
| Br | Br | H | $CH_3$ | H | $CH_3$ | $-CH_2-C{\equiv}CH$ |
| Cl | Cl | H | H | $CH_3$ | $CH_3$ | $-CH_2-C{\equiv}CH$ |
| Br | Br | H | H | $CH_3$ | $CH_3$ | $-CH_2-C{\equiv}CH$ |
| Cl | Cl | H | $CH_2Cl$ | H | H | $-CH_2-C{\equiv}CH$ |
| Br | Br | H | $CH_2Cl$ | H | H | $-CH_2-C{\equiv}CH$ |
| Cl | Cl | H | $CH_2Br$ | H | H | $-CH_2-C{\equiv}CH$ |
| Br | Br | H | $CH_2Br$ | H | H | $-CH_2-C{\equiv}CH$ |
| Cl | Cl | H | H | $CH_2Cl$ | H | $-CH_2-C{\equiv}CH$ |
| Br | Br | H | H | $CH_2Cl$ | H | $-CH_2-C{\equiv}CH$ |
| Cl | Cl | H | H | $CH_2Br$ | H | $-CH_2-C{\equiv}CH$ |
| Br | Br | H | H | $CH_2Br$ | H | $-CH_2-C{\equiv}CH$ |
| Cl | Cl | H | $H_2C{=}CH-CH_2OCH_2-$ | H | H | $-CH_2-C{\equiv}CH$ |
| Br | Br | H | $H_2C{=}CH-CH_2OCH_2-$ | H | H | $-CH_2-C{\equiv}CH$ |
| Cl | Cl | H | $HC{\equiv}C-CH_2OCH_2-$ | H | H | $-CH_2-C{\equiv}CH$ |
| Br | Br | H | $HC{\equiv}C-CH_2OCH_2-$ | H | H | $-CH_2-C{\equiv}CH$ |
| Cl | Cl | H | 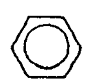 | H | H | $-CH_2-C{\equiv}CH$ |
| Br | Br | H | 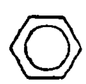 | H | H | $-CH_2-C{\equiv}CH$ |
| Cl | Cl | $CH_3$ | $CH_3$ | H | H | $-CH_2-C{\equiv}CH$ |
| Br | Br | $CH_3$ | $CH_3$ | H | H | $-CH_2-C{\equiv}CH$ |
| Cl | Cl | $CH_3$ | $CH_3$ | H | $CH_3$ | $-CH_2-C{\equiv}CH$ |
| Br | Br | $CH_3$ | $CH_3$ | H | $CH_3$ | $-CH_2-C{\equiv}CH$ |
| Cl | Cl | H | $CH_3$ | H | H | $-CH(CH_3)-C{\equiv}CH$ |
| Br | Br | H | $CH_3$ | H | H | $-CH(CH_3)-C{\equiv}CH$ |
| Cl | Cl | H | H | H | H | $-CH(CH_3)-C{\equiv}CH$ |
| Br | Br | H | H | H | H | $-CH(CH_3)-C{\equiv}CH$ |
| Cl | Cl | H | H | $CH_3$ | H | $-CH(CH_3)-C{\equiv}CH$ |
| Br | Br | H | H | $CH_3$ | H | $-CH(CH_3)-C{\equiv}CH$ |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_3$ | $CH_3$ | H | $-CH(CH_3)-C\equiv CH$ |
| Br | Br | H | $CH_3$ | $CH_3$ | H | $-CH(CH_3)-C\equiv CH$ |
| Cl | Cl | H | $CH_3$ | $CH_3$ | $CH_3$ | $-CH(CH_3)-C\equiv CH$ |
| Br | Br | H | $CH_3$ | $CH_3$ | $CH_3$ | $-CH(CH_3)-C\equiv CH$ |
| Cl | Cl | H | H | H | $CH_3$ | $-CH(CH_3)-C\equiv CH$ |
| Br | Br | H | H | H | $CH_3$ | $-CH(CH_3)-C\equiv CH$ |
| Cl | Cl | H | $CH_3$ | H | $CH_3$ | $-CH(CH_3)-C\equiv CH$ |
| Br | Br | H | $CH_3$ | H | $CH_3$ | $-CH(CH_3)-C\equiv CH$ |
| Cl | Cl | H | H | $CH_3$ | $CH_3$ | $-CH(CH_3)-C\equiv CH$ |
| Br | Br | H | H | $CH_3$ | $CH_3$ | $-CH(CH_3)-C\equiv CH$ |
| Cl | Cl | H | $CH_2Cl$ | H | H | $-CH(CH_3)-C\equiv CH$ |
| Br | Br | H | $CH_2Cl$ | H | H | $-CH(CH_3)-C\equiv CH$ |
| Cl | Cl | H | $CH_3Br$ | H | H | $-CH(CH_3)-C\equiv CH$ |
| Br | Br | H | $CH_2Br$ | H | H | $-CH(CH_3)-C\equiv CH$ |
| Cl | Cl | H | H | $CH_2Cl$ | H | $-CH(CH_3)-C\equiv CH$ |
| Br | Br | H | H | $CH_2Cl$ | H | $-CH(CH_3)-C\equiv CH$ |
| Cl | Cl | H | H | $CH_2Br$ | H | $-CH(CH_3)-C\equiv CH$ |
| Br | Br | H | H | $CH_2Br$ | H | $-CH(CH_3)-C\equiv CH$ |
| Cl | Cl | H | $H_2C=CH-CH_2OCH_2-$ | H | H | $-CH(CH_3)-C\equiv CH$ |
| Br | Br | H | $H_2C=CH-CH_2OCH_2-$ | H | H | $-CH(CH_3)-C\equiv CH$ |
| Cl | Cl | H | $HC\equiv C-CH_2OCH_2-$ | H | H | $-CH(CH_3)-C\equiv CH$ |
| Br | Br | H | $HC\equiv C-CH_2OCH_2-$ | H | H | $-CH(CH_3)-C\equiv CH$ |
| Cl | Cl | H | 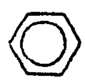 | H | H | $-CH(CH_3)-C\equiv CH$ |
| Br | Br | H | 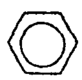 | H | H | $-CH(CH_3)-C\equiv CH$ |
| Cl | Cl | $CH_3$ | $CH_3$ | H | H | $-CH(CH_3)-C\equiv CH$ |
| Br | Br | $CH_3$ | $CH_3$ | H | H | $-CH(CH_3)-C\equiv CH$ |
| Cl | Cl | $CH_3$ | $CH_3$ | H | $CH_3$ | $-CH(CH_3)-C\equiv CH$ |
| Br | Br | $CH_3$ | $CH_3$ | H | $CH_3$ | $-CH(CH_3)-C\equiv CH$ |
| Cl | Cl | H | $CH_3$ | H | H | 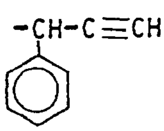 |
| Br | Br | H | $CH_3$ | H | H | 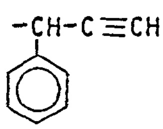 |
| Cl | Cl | H | H | H | H | 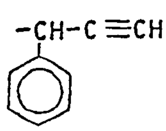 |
| Br | Br | H | H | H | H | 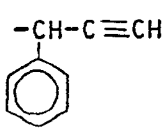 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | H | $CH_3$ | H | 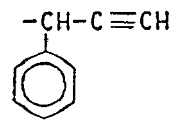 |
| Br | Br | H | H | $CH_3$ | H | 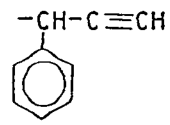 |
| Cl | Cl | H | $CH_3$ | $CH_3$ | H | 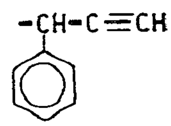 |
| Br | Br | H | $CH_3$ | $CH_3$ | H | 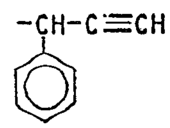 |
| Cl | Cl | H | $CH_3$ | $CH_3$ | $CH_3$ | 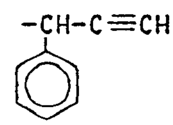 |
| Br | Br | H | $CH_3$ | $CH_3$ | $CH_3$ | 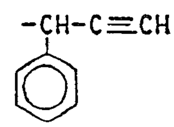 |
| Cl | Cl | H | H | H | $CH_3$ | 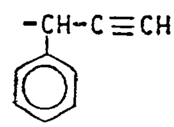 |
| Br | Br | H | H | H | $CH_3$ | 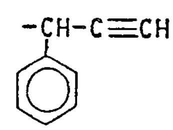 |
| Cl | Cl | H | $CH_3$ | H | $CH_3$ | 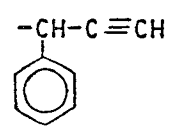 |
| Br | Br | H | $CH_3$ | H | $CH_3$ | 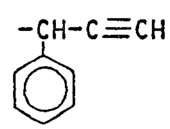 |
| Cl | Cl | H | H | $CH_3$ | $CH_3$ | 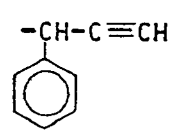 |
| Br | Br | H | H | $CH_3$ | $CH_3$ | 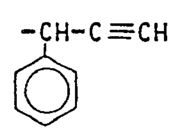 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_2Cl$ | H | H | 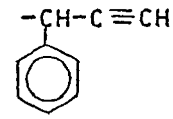 |
| Br | Br | H | $CH_2Cl$ | H | H | 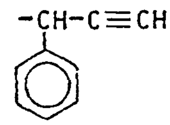 |
| Cl | Cl | H | $CH_2Br$ | H | H | 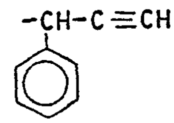 |
| Br | Br | H | $CH_2Br$ | H | H | 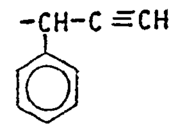 |
| Cl | Cl | H | H | $CH_2Cl$ | H | 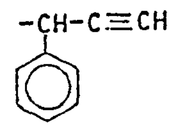 |
| Br | Br | H | H | $CH_2Cl$ | H | 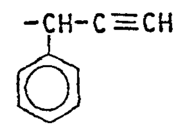 |
| Cl | Cl | H | H | $CH_2Br$ | H | 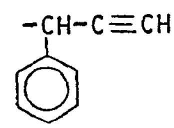 |
| Br | Br | H | H | $CH_2Br$ | H | 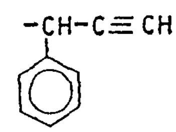 |
| Cl | Cl | H | $H_2C{=}CH{-}CH_2OCH_2{-}$ | H | H | 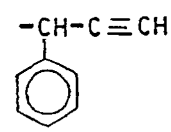 |
| Br | Br | H | $H_2C{=}CH{-}CH_2OCH_2{-}$ | H | H | 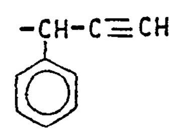 |
| Cl | Cl | H | $HC{\equiv}C{-}CH_2OCH_2{-}$ | H | H | 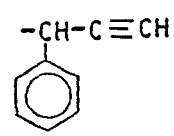 |
| Br | Br | H | $HC{\equiv}C{-}CH_2OCH_2{-}$ | H | H | 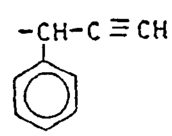 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | 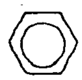 | H | H | 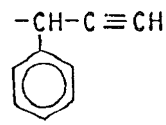 |
| Br | Br | H | 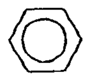 | H | H | 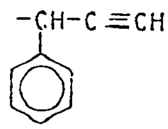 |
| Cl | Cl | $CH_3$ | $CH_3$ | H | H | 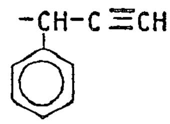 |
| Br | Br | $CH_3$ | $CH_3$ | H | H | 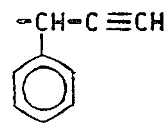 |
| Cl | Cl | $CH_3$ | $CH_3$ | H | $CH_3$ | 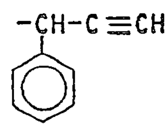 |
| Br | Br | $CH_3$ | $CH_3$ | H | $CH_3$ | 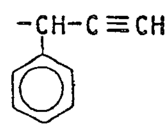 |
| Cl | Cl | H | $CH_3$ | H | H | $-CH(C_2H_5)-C{\equiv}CH$ |
| Br | Br | H | $CH_3$ | H | H | $-CH(C_2H_5)-C{\equiv}CH$ |
| Cl | Cl | H | H | H | H | $-CH(C_2H_5)-C{\equiv}CH$ |
| Br | Br | H | H | H | H | $-CH(C_2H_5)-C{\equiv}CH$ |
| Cl | Cl | H | H | $CH_3$ | H | $-CH(C_2H_5)-C{\equiv}CH$ |
| Br | Br | H | H | $CH_3$ | H | $-CH(C_2H_5)-C{\equiv}CH$ |
| Cl | Cl | H | $CH_3$ | $CH_3$ | H | $-CH(C_2H_5)-C{\equiv}CH$ |
| Br | Br | H | $CH_3$ | $CH_3$ | H | $-CH(C_2H_5)-C{\equiv}CH$ |
| Cl | Cl | H | $CH_3$ | $CH_3$ | $CH_3$ | $-CH(C_2H_5)-C{\equiv}CH$ |
| Br | Br | H | $CH_3$ | $CH_3$ | $CH_3$ | $-CH(C_2H_5)-C{\equiv}CH$ |
| Cl | Cl | H | H | H | $CH_3$ | $-CH(C_2H_5)-C{\equiv}CH$ |
| Br | Br | H | H | H | $CH_3$ | $-CH(C_2H_5)-C{\equiv}CH$ |
| Cl | Cl | H | $CH_3$ | H | $CH_3$ | $-CH(C_2H_5)-C{\equiv}CH$ |
| Br | Br | H | $CH_3$ | H | $CH_3$ | $-CH(C_2H_5)-C{\equiv}CH$ |
| Cl | Cl | H | H | $CH_3$ | $CH_3$ | $-CH(C_2H_5)-C{\equiv}CH$ |
| Br | Br | H | H | $CH_3$ | $CH_3$ | $-CH(C_2H_5)-C{\equiv}CH$ |
| Cl | Cl | H | $CH_2Cl$ | H | H | $-CH(C_2H_5)-C{\equiv}CH$ |
| Br | Br | H | $CH_2CL$ | H | H | $-CH(C_2H_5)-C{\equiv}CH$ |
| Cl | Cl | H | $CH_2Br$ | H | H | $-CH(C_2H_5)-C{\equiv}CH$ |
| Br | Br | H | $CH_2Br$ | H | H | $-CH(C_2H_5)-C{\equiv}CH$ |
| Cl | Cl | H | H | $CH_2Cl$ | H | $-CH(C_2H_5)-C{\equiv}CH$ |
| Br | Br | H | H | $CH_2Cl$ | H | $-CH(C_2H_5)-C{\equiv}CH$ |
| Cl | Cl | H | H | $CH_2Br$ | H | $-CH(C_2H_5)-C{\equiv}CH$ |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Br | Br | H | H | $CH_2Br$ | H | $-CH(C_2H_5)-C\equiv CH$ |
| Cl | Cl | H | $H_2C=CH-CH_2OCH_2-$ | H | H | $-CH(C_2H_5)-C\equiv CH$ |
| Br | Br | H | $H_2C=CH-CH_2OCH_2-$ | H | H | $-CH(C_2H_5)-C\equiv CH$ |
| Cl | Cl | H | $HC\equiv C-CH_2OCH_2-$ | H | H | $-CH(C_2H_5)-C\equiv CH$ |
| Br | Br | H | $HC\equiv C-CH_2OCH_2-$ | H | H | $-CH(C_2H_5)-C\equiv CH$ |
| Cl | Cl | H | 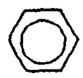 | H | H | $-CH(C_2H_5)-C\equiv CH$ |
| Br | Br | H | 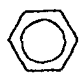 | H | H | $-CH(C_2H_5)-C\equiv CH$ |
| Cl | Cl | $CH_3$ | $CH_3$ | H | H | $-CH(C_2H_5)-C\equiv CH$ |
| Br | Br | $CH_3$ | $CH_3$ | H | H | $-CH(C_2H_5)-C\equiv CH$ |
| Cl | Cl | $CH_3$ | $CH_3$ | H | $CH_3$ | $-CH(C_2H_5)-C\equiv CH$ |
| Br | Br | $CH_3$ | $CH_3$ | H | $CH_3$ | $-CH(C_2H_5)-C\equiv CH$ |
| Cl | Cl | H | $CH_3$ | H | H | 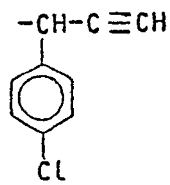 |
| Br | Br | H | $CH_3$ | H | H | 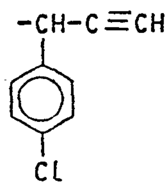 |
| Cl | Cl | H | H | H | H | 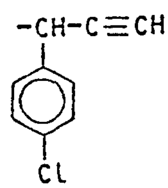 |
| Br | Br | H | H | H | H | 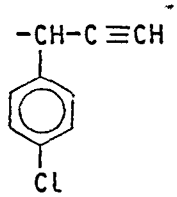 |
| Cl | Cl | H | H | $CH_3$ | H | 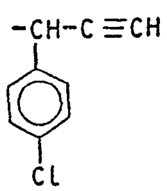 |
| Br | Br | H | H | $CH_3$ | H | 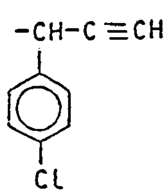 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_3$ | $CH_3$ | H | 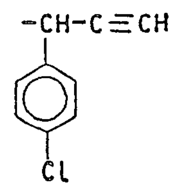 |
| Br | Br | H | $CH_3$ | $CH_3$ | H | 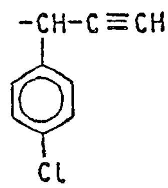 |
| Cl | Cl | H | $CH_3$ | $CH_3$ | $CH_3$ | 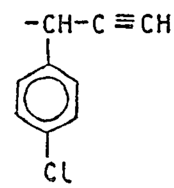 |
| Br | Br | H | $CH_3$ | $CH_3$ | $CH_3$ | 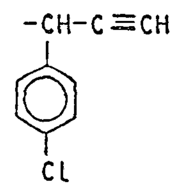 |
| Cl | Cl | H | H | H | $CH_3$ | 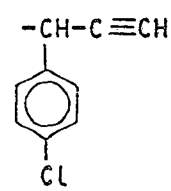 |
| Br | Br | H | H | H | $CH_3$ | 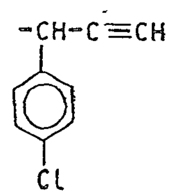 |
| Cl | Cl | H | $CH_3$ | H | $CH_3$ | 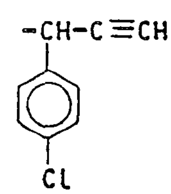 |
| Br | Br | H | $CH_3$ | H | $CH_3$ | 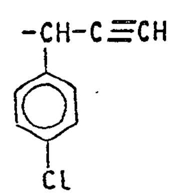 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | H | $CH_3$ | $CH_3$ | 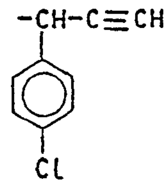 |
| Br | Br | H | H | $CH_3$ | $CH_3$ | 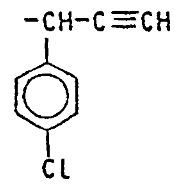 |
| Cl | Cl | H | $CH_2Cl$ | H | H | 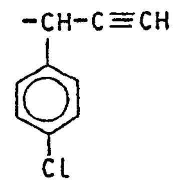 |
| Br | Br | H | $CH_2Cl$ | H | H | 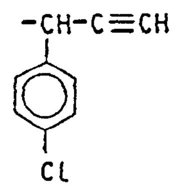 |
| Cl | Cl | H | $CH_2Br$ | H | H | 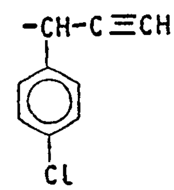 |
| Br | Br | H | $CH_2Br$ | H | H | 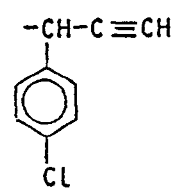 |
| Cl | Cl | H | H | $CH_2Cl$ | H | 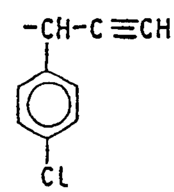 |
| Br | Br | H | H | $CH_2Cl$ | H | 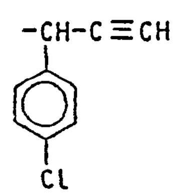 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | H | $CH_2Br$ | H | 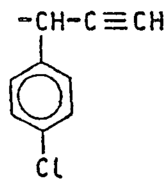 |
| Br | Br | H | H | $CH_2Br$ | H | 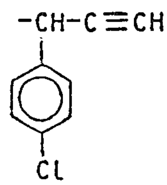 |
| Cl | Cl | H | $H_2C=CH-CH_2OCH_2-$ | H | H | 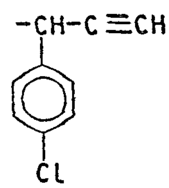 |
| Br | Br | H | $H_2C=CH-CH_2OCH_2-$ | H | H | 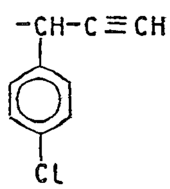 |
| Cl | Cl | H | $HC\equiv C-CH_2OCH_2-$ | H | H | 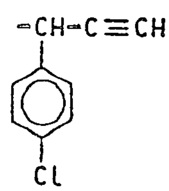 |
| Br | Br | H | $HC\equiv C-CH_2OCH_2-$ | H | H | 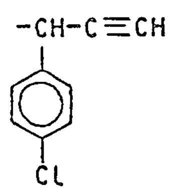 |
| Cl | Cl | H | 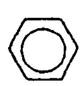 | H | H | 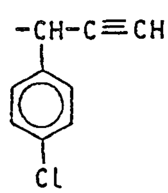 |
| Br | Br | H | 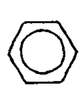 | H | H | 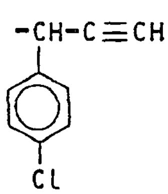 |

*Tabelle 3* (Fortsetzung)

| $X^1$ | $X^2$ | $R^1$ | $R^2$ | $R^3$ | $R^4$ | $-CR^5R^6R^7$ |
|---|---|---|---|---|---|---|
| Cl | Cl | $CH_3$ | $CH_3$ | H | H | 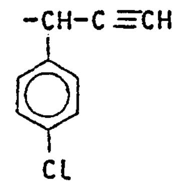 |
| Br | Br | $CH_3$ | $CH_3$ | H | H | 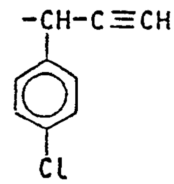 |
| Cl | Cl | $CH_3$ | $CH_3$ | H | $CH_3$ | 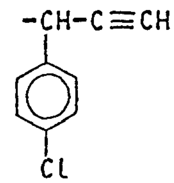 |
| Br | Br | $CH_3$ | $CH_3$ | H | $CH_3$ | 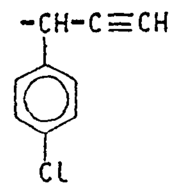 |
| Cl | Cl | H | $CH_3$ | H | H | 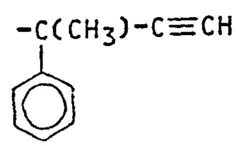 |
| Br | Br | H | $CH_3$ | H | H | 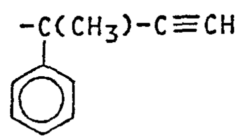 |
| Cl | Cl | H | H | H | H | 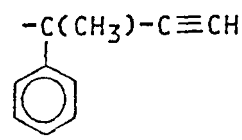 |
| Br | Br | H | H | H | H | 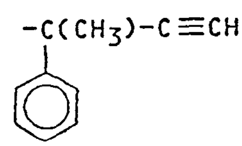 |
| Cl | Cl | H | H | $CH_3$ | H | 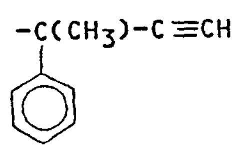 |
| Br | Br | H | H | $CH_3$ | H | 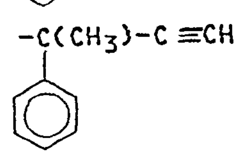 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_3$ | $CH_3$ | H | 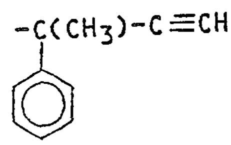 |
| Br | Br | H | $CH_3$ | $CH_3$ | H | 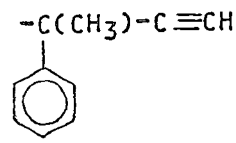 |
| Cl | Cl | H | $CH_3$ | $CH_3$ | $CH_3$ | 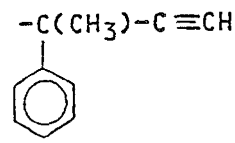 |
| Br | Br | H | $CH_3$ | $CH_3$ | $CH_3$ | 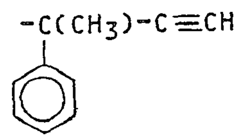 |
| Cl | Cl | H | H | H | $CH_3$ | 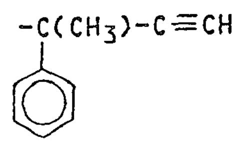 |
| Br | Br | H | H | H | $CH_3$ | 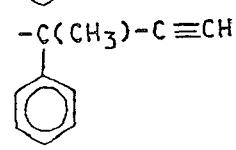 |
| Cl | Cl | H | $CH_3$ | H | $CH_3$ | 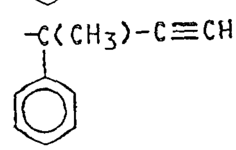 |
| Br | Br | H | $CH_3$ | H | $CH_3$ | 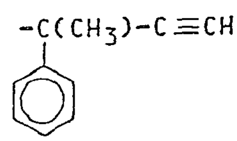 |
| Cl | Cl | H | H | $CH_3$ | $CH_3$ | 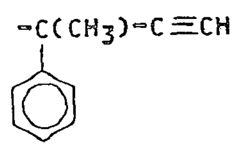 |
| Br | Br | H | H | $CH_3$ | $CH_3$ | 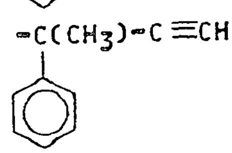 |
| Cl | Cl | H | $CH_2Cl$ | H | H | 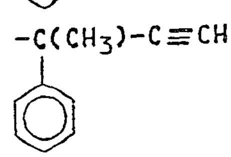 |
| Br | Br | $CH_3$ | $CH_2Cl$ | H | H | 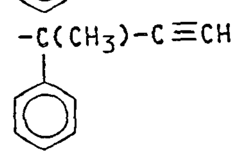 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_2Br$ | H | H | 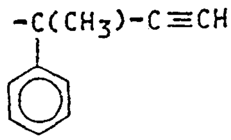 |
| Br | Br | H | $CH_2Br$ | H | H | 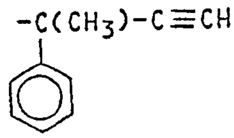 |
| Cl | Cl | H | H | $CH_2Cl$ | H | 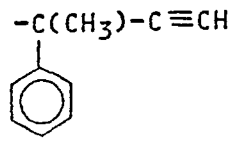 |
| Br | Br | H | H | $CH_2Cl$ | H | 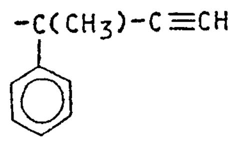 |
| Cl | Cl | H | H | $CH_2Br$ | H | 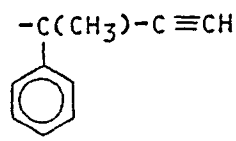 |
| Br | Br | H | H | $CH_2Br$ | H | 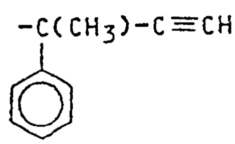 |
| Cl | Cl | H | $H_2C=CH-CH_2OCH_2-$ | H | H | 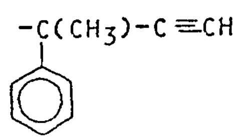 |
| Br | Br | H | $H_2C=CH-CH_2OCH_2-$ | H | H | 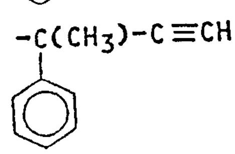 |
| Cl | Cl | H | $HC\equiv C-CH_2OCH_2-$ | H | H | 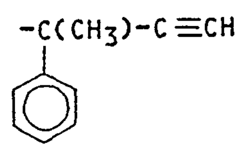 |
| Br | Br | H | $HC\equiv C-CH_{2o}OCH_2-$ | H | H | 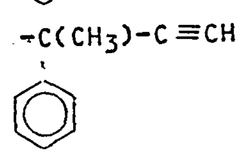 |
| Cl | Cl | H | 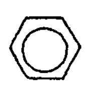 | H | H | 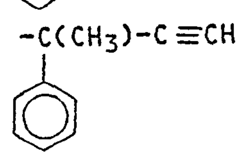 |
| Br | Br | H | 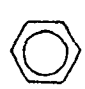 | H | H | 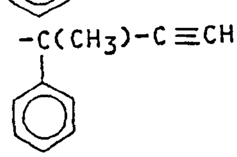 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | $CH_3$ | $CH_3$ | H | H | 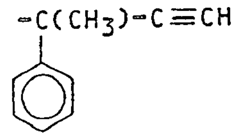 |
| Br | Br | $CH_3$ | $CH_3$ | H | H | 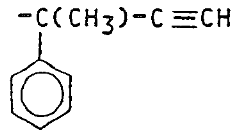 |
| Cl | Cl | $CH_3$ | $CH_3$ | H | $CH_3$ | 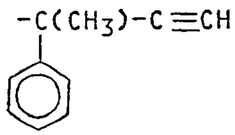 |
| Br | Br | $CH_3$ | $CH_3$ | H | $CH_3$ | 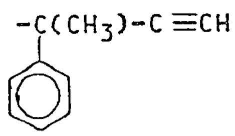 |
| Cl | Cl | H | $CH_3$ | H | H | $-CH_2-CH_2-C\equiv CH$ |
| Br | Br | H | $CH_3$ | H | H | $-CH_2-CH_2-C\equiv CH$ |
| Cl | Cl | H | H | H | H | $-CH_2-CH_2-C\equiv CH$ |
| Br | Br | H | H | H | H | $-CH_2-CH_2-C\equiv CH$ |
| Cl | Cl | H | H | $CH_3$ | H | $-CH_2-CH_2-C\equiv CH$ |
| Br | Br | H | H | $CH_3$ | H | $-CH_2-CH_2-C\equiv CH$ |
| Cl | Cl | H | $CH_3$ | $CH_3$ | H | $-CH_2-CH_2-C\equiv CH$ |
| Br | Br | H | $CH_3$ | $CH_3$ | H | $-CH_2-CH_2-C\equiv CH$ |
| Cl | Cl | H | $CH_3$ | $CH_3$ | $CH_3$ | $-CH_2-CH_2-C\equiv CH$ |
| Br | Br | H | $CH_3$ | $CH_3$ | $CH_3$ | $-CH_2-CH_2-C\equiv CH$ |
| Cl | Cl | H | H | H | $CH_3$ | $-CH_2-CH_2-C\equiv CH$ |
| Br | Br | H | H | H | $CH_3$ | $-CH_2-CH_2-C\equiv CH$ |
| Cl | Cl | H | $CH_3$ | H | $CH_3$ | $-CH_2-CH_2-C\equiv CH$ |
| Br | Br | H | $CH_3$ | H | $CH_3$ | $-CH_2-CH_2-C\equiv CH$ |
| Cl | Cl | H | H | $CH_3$ | $CH_3$ | $-CH_2-CH_2-C\equiv CH$ |
| Br | Br | H | H | $CH_3$ | $CH_3$ | $-CH_2-CH_2-C\equiv CH$ |
| Cl | Cl | H | $CH_2Cl$ | H | H | $-CH_2-CH_2-C\equiv CH$ |
| Br | Br | H | $CH_2Cl$ | H | H | $-CH_2-CH_2-C\equiv CH$ |
| Cl | Cl | H | $CH_2Br$ | H | H | $-CH_2-CH_2-C\equiv CH$ |
| Br | Br | H | $CH_2Br$ | H | H | $-CH_2-CH_2-C\equiv CH$ |
| Cl | Cl | H | H | $CH_2Cl$ | H | $-CH_2-CH_2-C\equiv CH$ |
| Br | Br | H | H | $CH_2Cl$ | H | $-CH_2-CH_2-C\equiv CH$ |
| Cl | Cl | H | H | $CH_2Br$ | H | $-CH_2-CH_2-C\equiv CH$ |
| Br | Br | H | H | $CH_2Br$ | H | $-CH_2-CH_2-C\equiv CH$ |
| Cl | Cl | H | $H_2C=CH-CH_2OCH_2-$ | H | H | $-CH_2-CH_2-C\equiv CH$ |
| Br | Br | H | $H_2C=CH-CH_2OCH_2-$ | H | H | $-CH_2-CH_2-C\equiv CH$ |
| Cl | Cl | H | $HC\equiv C-CH_2OCH_2-$ | H | H | $-CH_2-CH_2-C\equiv CH$ |
| Br | Br | H | $HC\equiv C-CH_2OCH_2-$ | H | H | $-CH_2-CH_2-C\equiv CH$ |
| Cl | Cl | H | 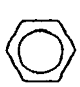 | H | H | $-CH_2-CH_2-C\equiv CH$ |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Br | Br | H | 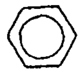 | H | H | $-CH_2-CH_2-C\equiv CH$ |
| Cl | Cl | $CH_3$ | $CH_3$ | H | H | $-CH_2-CH_2-C\equiv CH$ |
| Br | Br | $CH_3$ | $CH_3$ | H | H | $-CH_2-CH_2-C\equiv CH$ |
| Cl | Cl | $CH_3$ | $CH_3$ | H | $CH_3$ | $-CH_2-CH_2-C\equiv CH$ |
| Br | Br | $CH_3$ | $CH_3$ | H | $CH_3$ | $-CH_2-CH_2-C\equiv CH$ |
| Cl | Cl | H | $CH_3$ | H | H | $-CH(CH_3)-CH_2-C\equiv CH$ |
| Br | Br | H | $CH_3$ | H | H | $-CH(CH_3)-CH_2-C\equiv CH$ |
| Cl | Cl | H | H | H | H | $-CH(CH_3)-CH_2-C\equiv CH$ |
| Br | Br | H | H | H | H | $-CH(CH_3)-CH_2-C\equiv CH$ |
| Cl | Cl | H | H | $CH_3$ | H | $-CH(CH_3)-CH_2-C\equiv CH$ |
| Br | Br | H | H | $CH_3$ | H | $-CH(CH_3)-CH_2-C\equiv CH$ |
| Cl | Cl | H | $CH_3$ | $CH_3$ | H | $-CH(CH_3)-CH_2-C\equiv CH$ |
| Br | Br | H | $CH_3$ | $CH_3$ | H | $-CH(CH_3)-CH_2-C\equiv CH$ |
| Cl | Cl | H | $CH_3$ | $CH_3$ | $CH_3$ | $-CH(CH_3)-CH_2-C\equiv CH$ |
| Br | Br | H | $CH_3$ | $CH_3$ | $CH_3$ | $-CH(CH_3)-CH_2-C\equiv CH$ |
| Cl | Cl | H | H | H | $CH_3$ | $-CH(CH_3)-CH_2-C\equiv CH$ |
| Br | Br | H | H | H | $CH_3$ | $-CH(CH_3)-CH_2-C\equiv CH$ |
| Cl | Cl | H | $CH_3$ | H | $CH_3$ | $-CH(CH_3)-CH_2-C\equiv CH$ |
| Br | Br | H | $CH_3$ | H | $CH_3$ | $-CH(CH_3)-CH_2-C\equiv CH$ |
| Cl | Cl | H | H | $CH_3$ | $CH_3$ | $-CH(CH_3)-CH_2-C\equiv CH$ |
| Br | Br | H | H | $CH_3$ | $CH_3$ | $-CH(CH_3)-CH_2-C\equiv CH$ |
| Cl | Cl | H | $CH_2Cl$ | H | H | $-CH(CH_3)-CH_2-C\equiv CH$ |
| Br | Br | H | $CH_2Cl$ | H | H | $-CH(CH_3)-CH_2-C\equiv CH$ |
| Cl | Cl | H | $CH_2Br$ | H | H | $-CH(CH_3)-CH_2-C\equiv CH$ |
| Br | Br | H | $CH_2Br$ | H | H | $-CH(CH_3)-CH_2-C\equiv CH$ |
| Cl | Cl | H | H | $CH_2Cl$ | H | $-CH(CH_3)-CH_2-C\equiv CH$ |
| Br | Br | H | H | $CH_2Cl$ | H | $-CH(CH_3)-CH_2-C\equiv CH$ |
| Cl | Cl | H | H | $CH_2Br$ | H | $-CH(CH_3)-CH_2-C\equiv CH$ |
| Br | Br | H | H | $CH_2Br$ | H | $-CH(CH_3)-CH_2-C\equiv CH$ |
| Cl | Cl | H | $H_2C=CH-CH_2OCH_2-$ | H | H | $-CH(CH_3)-CH_2-C\equiv CH$ |
| Br | Br | H | $H_2C=CH-CH_2OCH_2-$ | H | H | $-CH(CH_3)-CH_2-C\equiv CH$ |
| Cl | Cl | H | $HC\equiv C-CH_2OCH_2-$ | H | H | $-CH(CH_3)-CH_2-C\equiv CH$ |
| Br | Br | H | $HC\equiv C-CH_2OCH_2-$ | H | H | $-CH(CH_3)-CH_2-C\equiv CH$ |
| Cl | Cl | H | 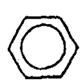 | H | H | $-CH(CH_3)-CH_2-C\equiv CH$ |
| Br | Br | H | 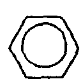 | H | H | $-CH(CH_3)-CH_2-C\equiv CH$ |
| Cl | Cl | $CH_3$ | $CH_3$ | H | H | $-CH(CH_3)-CH_2-C\equiv CH$ |
| Br | Br | $CH_3$ | $CH_3$ | H | H | $-CH(CH_3)-CH_2-C\equiv CH$ |
| Cl | Cl | $CH_3$ | $CH_3$ | H | $CH_3$ | $-CH(CH_3)-CH_2-C\equiv CH$ |
| Br | Br | $CH_3$ | $CH_3$ | H | $CH_3$ | $-CH(CH_3)-CH_2-C\equiv CH$ |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_3$ | H | H | 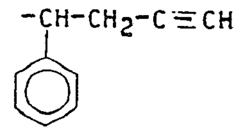 |
| Br | Br | H | $CH_3$ | H | H | 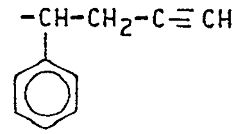 |
| Cl | Cl | H | H | H | H | 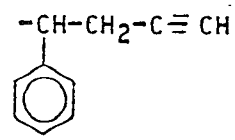 |
| Br | Br | H | H | H | H | 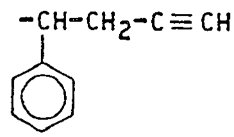 |
| Cl | Cl | H | H | $CH_3$ | H | 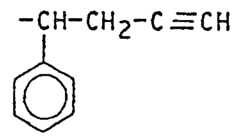 |
| Br | Br | H | H | $CH_3$ | H | 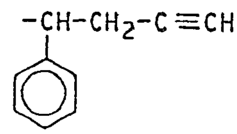 |
| Cl | Cl | H | $CH_3$ | $CH_3$ | H | 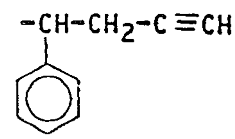 |
| Br | Br | H | $CH_3$ | $CH_3$ | H | 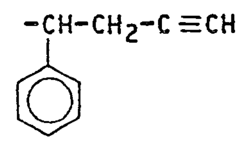 |
| Cl | Cl | H | $CH_3$ | $CH_3$ | $CH_3$ | 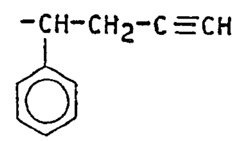 |
| Br | Br | H | $CH_3$ | $CH_3$ | $CH_3$ | 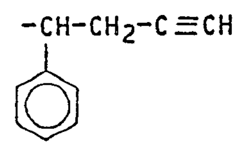 |
| Cl | Cl | H | H | H | $CH_3$ | 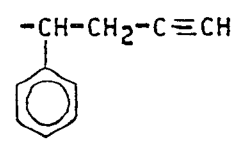 |
| Br | Br | H | H | H | $CH_3$ | 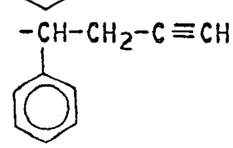 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $CH_3$ | H | $CH_3$ | 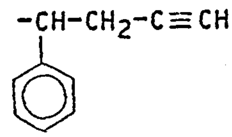 |
| Br | Br | H | $CH_3$ | H | $CH_3$ | 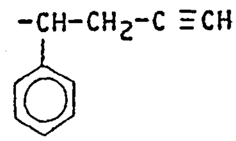 |
| Cl | Cl | H | H | $CH_3$ | $CH_3$ | 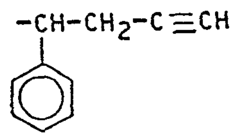 |
| Br | Br | H | H | $CH_3$ | $CH_3$ | 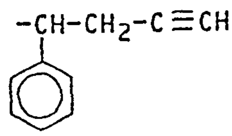 |
| Cl | Cl | H | $CH_2Cl$ | H | H | 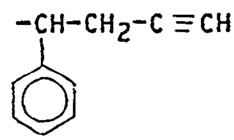 |
| Br | Br | H | $CH_2Cl$ | H | H | 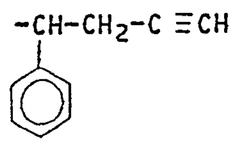 |
| Cl | Cl | H | $CH_2Br$ | H | H | 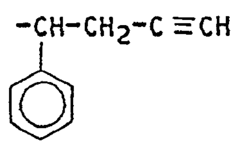 |
| Br | Br | H | $CH_2Br$ | H | H | 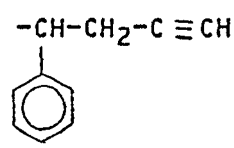 |
| Cl | Cl | H | H | $CH_2Cl$ | H | 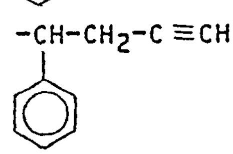 |
| Br | Br | H | H | $-CH_2Cl$ | H | 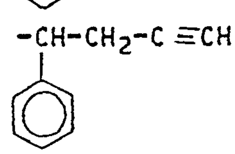 |
| Cl | Cl | H | H | $CH_2Br$ | H | 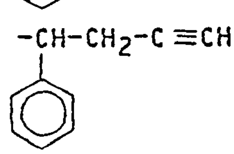 |
| Br | Br | H | H | $-CH_2Br$ | H | 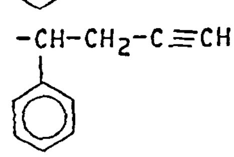 |

*Tabelle 3* (Fortsetzung)

| $X^1$ | $X^2$ | $R^1$ | $R^2$ | $R^3$ | $R^4$ | $-CR^5R^6R'$ |
|---|---|---|---|---|---|---|
| Cl | Cl | H | $H_2C{=}CH{-}CH_2OCH_2{-}$ | H | H | 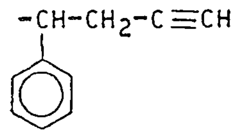 |
| Br | Br | H | $H_2C{=}CH{-}CH_2OCH_2{-}$ | H | H | 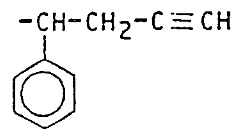 |
| Cl | Cl | H | $HC{\equiv}C{-}CH_2OCH_2{-}$ | H | H | 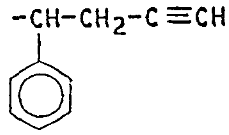 |
| Br | Br | H | $HC{\equiv}C{-}CH_2OCH_2{-}$ | H | H | 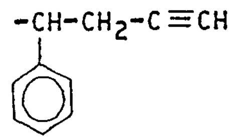 |
| Cl | Cl | H | 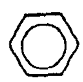 | H | H | 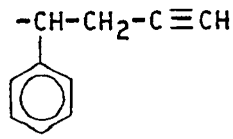 |
| Br | Br | H | 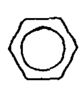 | H | H | 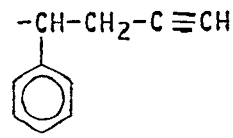 |
| Cl | Cl | $CH_3$ | $CH_3$ | H | H | 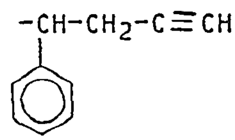 |
| Br | Br | $CH_3$ | $CH_3$ | H | H | 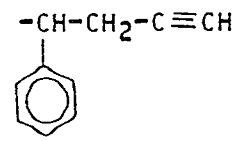 |
| ClH | Cl | $CH_3$ | $CH_3$ | H | $CH_3$ | 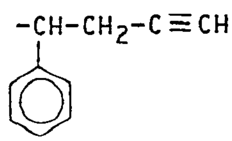 |
| Br | Br | $CH_3$ | $CH_3$ | H | $CH_3$ | 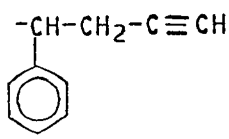 |

Die neuen Cyclopropylmethyl(en)ether der Formel (I) weisen in Mischung mit Stoffen beliebiger Konstitution, welche gegen Arthropoden wirksam sind, starke synergistische Wirkungen auf, welches ihre Verwendung in Schädlingsbekämpfungsmitteln zur Bekämpfung von tierischen Schädlingen ermöglicht.

Bevorzugt werden die neuen Cyclopropylmethyl(en)ether der Formel (I) zusammen mit Arthropodiziden der Gruppen

A) Carbaminsäureestern, und/oder

B) Carbonsäureestern einschliesslich der natürlichen sowie synthetischen Pyrethroide, und/oder

C) Phosphorverbindungen, wie Phosphorsäure- und Phosphonsäureestern, einschliesslich der Thio- und Dithioverbindungen.

Überraschenderweise ist die Wirkung der neuen erfindungsgemässen Wirkstoffkombinationen gegen Arthropoden wesentlich höher als die Wirkung der Einzelkomponenten bzw. die Summe der Wirkungen der Einzelkomponenten. Sie ist ferner wesentlich höher als die Wirkung von Wirkstoffkombinationen mit dem bekannten Synergisten Piperonylbutoxid. Die erfindungsgemässen Cyclopropylmethyl(en)ether der Formel (I) weisen diese ausgezeichnete synergistische Wirksamkeit nicht nur bei einer Wirkstoffklasse, sondern bei Wirkstoffen aus den verschiedensten chemischen Stoffgruppen auf. Die erfindungsgemässen Stoffe der Formel (I) besitzen ausserdem eine sehr gute bakterizide Wirkung und können zusätzlich auch zur Bekämpfung von Venturia-Arten wie z.B. gegen den Erreger von Apfelschorf *(Venturia inaequalis)* eingesetzt werden.

Die synergistische Wirkung der Verbindungen der Formel (I) zeigt sich besonders bevorzugt bei (in den folgenden Formeln wurden die Symbole $R^7$, $R^8$ und $R^9$ für eine evtl. weitere Verwendung unbenutzt belassen)

A) Carbaminsäureestern der Formel (IV)

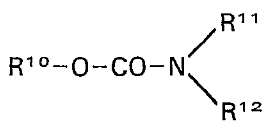 (IV)

in welcher

$R^{10}$ für einen gegebenenfalls substituierten carbocyclischen oder heterocyclischen aromatischen Rest oder für einen gegebenenfalls substituierten Oximrest steht (wobei die weiter unten erläuterten Reste $R^{10}$ bevorzugt werden),

$R^{11}$ für $C_1$-$C_4$-Alkyl steht, und

$R^{12}$ für Wasserstoff, $C_1$-$C_4$-Alkyl oder für einen Rest Y steht, wobei

Y für den Rest $-CO-R^{13}$ steht, worin

$R^{13}$ für Halogen, $C_1$-$C_4$-Alkyl, $C_1$-$C_4$-Alkoxy, $C_3$-$C_5$-Alkenoxy, $C_3$-$C_5$-Alkinoxy, $C_1$-$C_4$-Alkylthio, $C_1$-$C_4$-Alkylamino, Di-$C_1$-$C_4$-Alkylamino, $C_1$-$C_4$-Alkylhydroxylamino,
für gegebenenfalls durch Halogen, Nitro, Cyano, Trifluormethyl, $C_1$-$C_4$-Alkyl, $C_1$-$C_4$-Alkoxy, $C_1$-$C_4$-Alkylendioxy, $C_1$-$C_4$-Alkylthio, $C_1$-$C_4$-Alkoxycarbonyl substituiertes Phenoxy, Phenylthio oder Phenylamino, für 2,3-Dihydro-2,2-dimethyl-7-benzofuranyl oder für den Rest

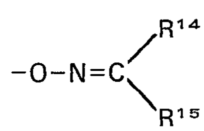

steht,
worin

$R^{14}$ für Wasserstoff, $C_1$-$C_4$-Alkyl oder Di-$C_1$-$C_4$-alkylaminocarbonyl steht, und

$R^{15}$ für $C_1$-$C_4$-Alkyl, $C_1$-$C_4$-Alkylthio, Cyano-$C_1$-$C_4$-alkylthio, $C_1$-$C_4$-Alkylthio-$C_1$-$C_4$-alkyl steht,

oder die beiden Reste $R^{14}$ und $R^{15}$ zusammen für gegebenenfalls durch Sauerstoff, Schwefel, SO oder $SO_2$ unterbrochenes $C_2$-$C_8$-Alkandiyl stehen, oder in welcher

Y für den Rest $-S_n(O)_m-R^{16}$ steht, worin

n für 1 oder 2, und

m für 0, 1 oder 2 stehen, und

$R^{16}$ für gegebenenfalls durch Halogen substituiertes $C_1$-$C_4$-Alkyl, $C_3$-$C_5$-Alkenyl, $C_3$-$C_5$-Alkinyl oder $C_3$-$C_6$-Cycloalkyl, für gegebenenfalls durch Halogen, Cyano, Nitro, Trifluormethyl, Trifluormethoxy, Trifluormethylthio, $C_1$-$C_4$-Alkyl oder $C_1$-$C_4$-Alkoxy substituiertes Phenyl, Benzyl oder Phenylethyl oder für den Rest

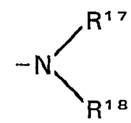

steht,
worin

$R^{17}$ für $C_1$-$C_4$-Alkyl, $C_3$-$C_5$-Alkenyl, $C_3$-$C_5$-Alkinyl, $C_3$-$C_6$-Cycloalkyl oder Benzyl steht, und

$R^{18}$ für $C_1$-$C_4$-Alkyl, $C_3$-$C_5$-Alkenyl, $C_3$-$C_5$-Alkinyl, $C_3$-$C_6$-Cycloalkyl, Benzyl, Phenylethyl, Halogencarbonyl, Formyl, $C_1$-$C_4$-Alkylcarbonyl, $C_1$-$C_4$-Alkoxycarbonyl, $C_1$-$C_4$-Alkoxyphenoxycarbonyl, $C_3$-$C_5$-Alkinoxycarbonyl, $C_3$-$C_5$-Alkenyloxycarbonyl, $C_1$-$C_4$-Alkylthiocarbonyl, $C_1$-$C_4$-Alkylaminocarbonyl, $C_1$-$C_4$-Alkylhydroxylaminocarbonyl, $C_1$-$C_4$-Alkylphenoxycarbonyl, Di-$C_1$-$C_4$-alkylaminocarbonyl, Phenylthiocarbonyl, Phenoxycarbonyl, 2,3-Dihydro-2,2-dimethyl-7-benzofuranyloxycarbonyl, für gegebenenfalls durch Halogen, Cyano, Nitro, Trifluormethyl, $C_1$-$C_{10}$-Alkyl oder $C_1$-$C_4$-Alkoxy substituiertes Phenylsulfenyl, Phenylsulfinyl, Phenylsulfonyl oder Phenyl steht, oder für den Rest

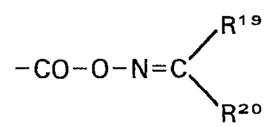

steht,
worin

$R^{19}$ die oben für $R^{14}$ angegebene Bedeutung hat, und

$R^{20}$ die oben für $R^{15}$ angegebene Bedeutung hat,

wobei ferner im Rest

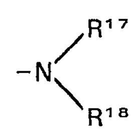

die Reste $R^{17}$ und $R^{18}$
zusammen für eine gegebenenfalls durch Sauerstoff oder Schwefel unterbrochene Kohlenwasserstoffkette mit 3 bis 8 Kohlenstoffatomen stehen, worin weiter $R^{16}$ auch für den gleichen Rest stehen kann, an den der Rest $-S_n(O)_m-R^{16}$ gebunden ist.

Als Wirkstoffkomponenten ganz besonders bevorzugt sind Carbaminsäureester der Formel (IV), in welcher

$R^{10}$ für gegebenenfalls durch $C_1$-$C_4$-Alkyl, $C_2$-$C_4$-Alkenyl, $C_1$-$C_4$-Alkoxy, $C_1$-$C_4$-Alkoxymethyl, $C_1$-$C_4$-Alkylthio, $C_1$-$C_4$-Alkylthiomethyl, $C_1$-$C_4$-Alkylamino, Di-($C_1$-$C_4$-alkyl)amino, Di-($C_3$-$C_4$-alkenyl)amino, Halogen, Dioxolanyl, Methylendioxy, Dimethyl-methylendioxy, und/oder durch den Rest $-N{=}CH(CH_3)_2$ substituierte Reste aus der Reihe Phenyl, Naphthyl, 2,3-Dihydro-7-benzofuranyl, Pyrazolyl oder Pyrimidinyl steht, oder

in welcher

$R^{10}$ für einen Alkylidenaminorest der Formel (IVa)

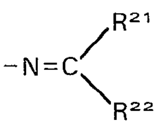 (IVa)

steht,

in welcher

$R^{21}$ und $R^{22}$ die oben für $R^{14}$ bzw. $R^{15}$ angegebene Bedeutung haben, und

$R^{11}$ für $C_1$-$C_4$-Alkyl steht, und

$R^{12}$ für Wasserstoff oder $C_1$-$C_4$-Alkyl (vorzugsweise für Wasserstoff) steht.

Als Beispiele für die Carbaminsäureester der Formel (IV) seien genannt: 2-Methylphenyl-, 2-Ethylphenyl-, 2-iso-Propylphenyl-, 2-sek.-Butylphenyl-, 2-Methoxyphenyl-, 2-Ethoxyphenyl-, 2-iso-Propoxyphenyl-, 4-Methylphenyl-, 4-Ethylphenyl-, 4-n-Propylphenyl-, 4-Methoxyphenyl-, 4-Ethoxyphenyl-, 4-n-Propoxyphenyl-, 3,4,5-Trimethylphenyl-, 3,5-Dimethyl-4-methylthiophenyl-, 3-Methyl-4-dimethylaminophenyl-, 2-Ethylthiomethylphenyl-, 1-Naphthyl-, 2,3-Dihydro-2,2-dimethyl-7-benzofuranyl, 2,3-(Dimethylmethylendioxy)phenyl-, 2-(1,3-Dioxolan-2-yl)-phenyl-, 2-(4,5-Dimethyl-1,3-dioxolan-2-yl)-phenyl-, 1-Methylthioethylidenamino-, 2-Methylthio-2-methylpropylidenamino-, 1-(2-Cyanoethylthio)ethylidenamino- und 1-Methylthiomethyl-2,2-dimethylpropylidenamino-N-methylcarbaminsäureester.

Die synergistische Wirkung der Verbindungen der Formel (I) zeigt sich weiter bevorzugt bei:

B) Carbonsäureestern der Formel (V)

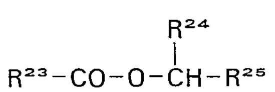 (V)

in welcher

$R^{23}$ für einen offenkettigen oder cyclischen Alkylrest steht, der gegebenenfalls substituiert ist durch Halogen, Alkyl, Cycloalkyl, durch gegebenenfalls durch Halogen, Alkyl und/oder Alkoxy substituiertes Alkenyl, durch Phenyl oder Styryl, welche gegebenenfalls durch Halogen, gegebenenfalls halogensubstituierte Reste aus der Reihe Alkyl, Alkoxy, Alkylendioxy und/oder Alkylthio substituiert sind, durch spirocyclisch verknüpftes, gegebenenfalls halogensubstituiertes Cycloalk(en)yl, welches gegebenenfalls benzanelliert ist, in welcher weiter

$R^{24}$ für Wasserstoff, Alkyl, Halogenalkyl, Alkenyl, Alkinyl oder Cyano steht, und

$R^{25}$ für einen gegebenenfalls substituierten Alkyl- oder Arylrest oder für einen Heterozyclus steht, oder zusammen mit $R^{24}$ und dem Kohlenstoffatom, an das beide Reste gebunden sind, einen Cyclopentenonring bildet.

Ganz besonders als Wirkstoffkomponenten bevorzugt sind Carbonsäureester der Formel (V), in welcher

$R^{23}$ für den Rest

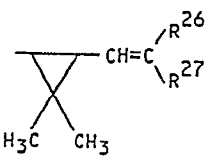

steht,

worin

$R^{26}$ für Wasserstoff, Methyl, Fluor, Chlor oder Brom, und

$R^{27}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls halogensubstituierte Reste der Reihe $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht, oder worin beide Reste $R^{26}$ und $R^{27}$ für $C_2$-$C_5$-Alkandiyl (Alkylen) stehen; oder

in welcher

$R^{23}$ für den Rest

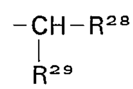

steht,

worin

$R^{28}$ für gegebenenfalls durch Halogen und/oder durch gegebenenfalls halogensubstituierte Reste der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht, und

$R^{29}$ für Isopropyl oder Cyclopropyl steht;

oder in welcher $R^{23}$ für einen der Reste

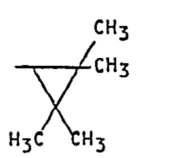

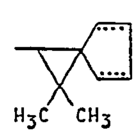

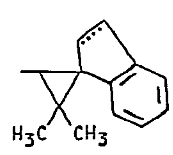

wobei die gepunkteten Linien mögliche Doppelbindungen andeuten sollen,

oder für Methyl steht,

und in welcher weiter

$R^{24}$ für Wasserstoff, $C_1$-$C_4$-Alkyl, $C_1$-$C_4$-Halogenalkyl, Cyano oder Ethinyl steht, und

$R^{25}$ gegebenenfalls durch Halogen substituierte Reste der Reihe Phenyl, Furyl oder Tetrahydrophthalimido steht, wobei diese Reste weiterhin substituiert sein können durch gegebenenfalls

durch Halogen und/oder durch einen gegebenenfalls halogensubstituierten Rest der Reihe $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl, wobei für $R^{25}$ vorzugsweise Pentafluorphenyl, Benzylfuryl, Phenoxyphenyl, welches in einem oder beiden Phenylringen durch Halogen substituiert sein kann, 3,4-Dichlorphenyl oder Tetrahydrophthalimido steht.

Weiter sind die natürliche vorkommenden Pyrethroide (wie Pyrethrum) als Carbonsäureester der Formel (V) besonders bevorzugt.

Als Beispiele für die Carbonsäureester der Formel (V) seien genannt:

Essigsäure-(2,2,2-trichlor-1-[3,4-dichlorphenyl]ethyl)ester, 2,2-Dimethyl-3-(2-methylpropen-1-yl)cyclopropancarbonsäure-(3,4,5,6-tetrahydrophthalimidomethyl)ester, 2,2-Dimethyl-3-(2,2-dichlorvinyl)cyclopropancarbonsäure-(3-phenoxybenzyl)ester, 2,2-Dimethyl-3-(2,2-dichlorvinyl)cyclopropancarbonsäure(α-cyano-3-phenoxybenzyl)ester, 2,2-Dimethyl-3-(2,2-dichlorvinyl)cyclopropancarbonsäure-(α-cyano-4-fluor-3-phenoxybenzyl)ester, 2,2-Dimethyl-3-(2,2-dichlorvinyl)-cyclopropancarbonsäure(pentafluorbenzyl)ester, 2,2-Dimethyl-3-(2,2-dibromvinyl)cyclopropancarbonsäure-(α-cyano-3-phenoxybenzyl)ester, 3-Methyl-2-(4-chlorphenyl)-butansäure-(α-cyano-3-phenoxybenzyl)ester und 2,2-Dimethyl-3-(2-methylpropen-1-yl)cyclopropancarbonsäure-(5-benzyl-3-furylmethyl)ester.

Weiter zeigt sich die synergistische Wirkung der Verbindungen der allgemeinen Formel (I) bevorzugt bei

C) Phosphorsäure- und Phosphonsäureestern der allgemeinen Formel (VI)

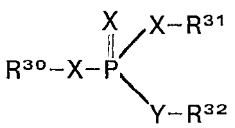

(VI)

in welcher

X jeweils für O oder S steht, und

Y für O, S, $-NH-$ oder für eine direkte Bindung zwischen dem zentralen P-Atom und $R^{32}$ steht, und

$R^{30}$ und $R^{31}$ gleich oder verschieden sind und für gegebenenfalls substituiertes Alkyl oder Aryl stehen,

$R^{32}$ für Wasserstoff, gegebenenfalls substituiertes Alkyl, Aryl, Heteroaryl, Aralkyl, Alkenyl, Dioxanyl oder einen Oximrest oder für den gleichen Rest steht, an den es gebunden ist.

Besonders bevorzugt sind Phosphorsäure- und Phosphonsäureester der Formel (VI), in welcher

$R^{30}$ und $R^{31}$ gleich oder verschieden sind und für $C_1$-$C_4$-Alkyl oder Phenyl stehen,

$R^{32}$ für Wasserstoff, Alkyl mit 1 bis 4 Kohlenstoffatomen steht, das gegebenenfalls durch Halogen, Hydroxyl, Cyano, gegebenenfalls halogensubstituiertes Phenyl, Carbamoyl, Alkylsulfonyl, Alkylsulfinyl, Alkylcarbonyl, Alkoxy, Alkylmercapto, Alkoxycarbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen, gegebenenfalls Halogen-substituiertes Phenyl oder $C_1$-$C_4$-Alkoxycarbonyl substituiert ist, oder für den Rest der allgemeinen Formel (VIa)

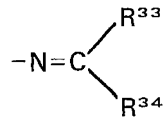

(VIa)

wobei $R^{33}$ und $R^{34}$ die oben für $R^{14}$ bzw. $R^{15}$ angegebene Bedeutung besitzen, oder für Cyano oder Phenyl stehen, und in welcher

$R^{32}$ ferner für Dioxanyl, das durch denselben Rest substituiert ist, an den $R^{32}$ gebunden ist, oder $R^{32}$ für den gleichen Rest, an den es gebunden ist, oder $R^{32}$ für Phenyl, das gegebenenfalls durch Methyl, Nitro, Cyano, Halogen und/oder Methylthio substituiert ist steht und $R^{32}$ ausserdem besonders bevorzugt für gegebenenfalls durch $C_1$-$C_4$-Alkoxy, $C_1$-$C_4$-Alkylthiomethyl, $C_1$-$C_4$-Alkyl und/oder Halogen-substituierte heteroaromatische Reste, wie Pyridinyl, Chinolinyl, Chinoxalinyl, Pyrimidinyl oder Benzo-1,2,4-triazinyl steht.

Im einzelnen seien genannt:

O,O-Dimethyl- bzw. O,O-Diethyl-O-(2,2-dichlor- bzw. 2,2-dibromvinyl)phosphorsäureester,
O,O-Diethyl-O-(4-nitrophenyl)thionophosphorsäureester,
O,O-Dimethyl-O-(3-methyl-4-methylthiophenyl)thionophosphorsäureester,
O,O-Dimethyl-O-(3-methyl-4-nitrophenyl)thionophosphorsäureester,
O-Ethyl-S-n-propyl-O-(2,4-dichlorphenyl)thionophosphorsäureester,
O-Ethyl-S-n-propyl-O-(4-methylthiophenyl)-thionophosphorsäureester,
O,O-Dimethyl-S-(4-oxo-1,2,3-benzotriazin-(3)ylmethyl)thionothiolphosphorsäureester,
O-Methyl-O-(2-iso-propyl-6-methoxypyrimidin(4)yl)thionomethanphosphonsäureester,
O,O-Diethyl-O-(2-iso-propyl-6-methylpyrimidin(4)yl)thionophosphorsäureester,
O,O-Diethyl-O-(3-chlor-4-methylcumarin(7)-yl)thionophosphorsäureester,
O,O-Dimethyl-2,2,2-trichlor-1-hydroxyethanphosphonsäureester,
O,O-Dimethyl-S-(methylaminocarbonylmethyl)-thionophosphorsäureester.

Die Gewichstsverhältnisse der Synergisten und Wirkstoffe können in einem relativ grossen Bereich variiert werden. Im allgemeinen werden die als Synergisten verwendeten Verbindungen der Formel (I) mit den übrigen Wirkstoffen in Mischungsverhältnissen zwischen 1:100 und 100:1, vorzugsweise zwischen 1:5 und 5:1 (Gewichtsteile) eingesetzt.

Die erfindungsgemässen Wirkstoffkombinationen besitzen nicht nur eine schnelle knock-down-Wirkung, sondern bewirken auch die nachhaltige Abtötung der tierischen Schädlinge, insbesondere von Insekten und Milben, die in der Landwirtschaft, in Forsten, im Vorrats- und Materialschutz sowie im Hygienebereich vorkommen. Sie sind gegen normal sensible und resistente Arten sowie gegen alle oder einzelne Entwicklungsstadien wirksam.

Zu den tierischen Schädlingen, welche unter Verwendung der Verbindungen der Formel (I) bekämpft werden können, gehören beispielsweise:

Aus der Ordnung der Isopoda z.B. *Oniscus asellus, Porcellio scaber.*

Aus der Ordnung der Thysanura z.B. *Lepisma saccharina.* Aus der Ordnung der Orthoptera z.B. *Blatta orientalis, Periplaneta americana, Leucophaea maderae, Blattella germanica, Acheta domesticus, Gryllotalpa spp., Locusta migratoria migratorioides, Schistocerca gregaria.* Aus der Ordnung der Dermaptera z.B. *Forficula auricularia.*

Aus der Ordnung der Isoptera z.B. *Reticulitermes spp.* Aus der Ordnung der Anoplura z.B. *Pediculus humanus corporis, Haematopinus spp., Linograthus spp.*

Aus der Ordnung der Mallophaga z.B. *Trichodectes spp., Damalinea spp.*

Aus der Ordnung der Heteroptera z.B. *Cimex lectularius, Rhodnius prolixus, Triatoma spp.*

Aus der Ordnung der Homoptera z.B. *Myzus spp.* und *Psylla spp.*

Aus der Ordnung der Lepidoptera z.B. *Ephestia kuehniella* und *Galleria mellonella.*

Aus der Ordnung der Coleoptera z.B. *Anobium punctatum, Rhizopertha dominica, Bruchidius obtectus, Acanthoscelides obtectus, Hylotrupes bajulus, Oryzaephilus surinamensis, Sitophilus spp., Dermestes spp., Trogoderma spp., Anthrenus spp., Attagenus spp., Lyctus spp., Ptinus spp., Niptus hololeucus, Gibbium psylloides, Tribolium spp.* und *Tenebrio molitor.*

Aus der Ordnung der Hymenoptera z.B. *Lasius spp., Monomorium pharaonis, Vespa spp.*

Aus der Ordnung der Diptera z.B. *Aädes spp., Anophelea spp., Culex spp., Drosophila melanogaster, Musca spp., Fannia spp., Calliphora erythrocephala, Lucilia spp., Chrysomyia spp., Gastrophilus spp., Hyppobosca spp., Stomoxys spp., Oestrus spp., Hypoderma spp.* und *Tabanus spp.*

Aus der Ordnung der Siphonaptera z.B. *Xenopsylla cheopis, Ceratophyllus spp.*

Aus der Ordnung der Arachnida z.B. *Scorpio maurus, Latrodectus mactans.*

Aus der Ordnung der Acarina z.B. *Acarus siro, Argas spp., Ornithodoros spp., Dermanyssus gallinae, Boophilus spp., Rhipicephalus spp., Amblyomma spp., Hyalomma spp., Ixodes spp., Psoroptes spp., Chorioptes spp., Sarcoptes spp.*

Die Wirkstoffkombinationen aus den Verbindungen der Formel (I) und den übrigen Wirkstoffen können in die üblichen Formulierungen übergeführt werden, wie Lösungen, Emulsionen, Spritzpulver, Suspensionen, Pulver, Stäubemittel, Schäume, Pasten, lösliche Pulver, Aerosole, Suspensions-Emulsionskonzentrate, wirkstoffimprägnierte Natur- und synthetische Stoffe, Feinstverkapselungen in polymeren Stoffen, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffgemische mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel: nichtionogene und anionische Emulgatoren, wie Polyoxyethylenfettsäureester, Polyoxyethylenfettalkoholether, z.B. Alkylarylpolyglykolether, Alkylsulfonate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoffkombination, vorzugsweise zwischen 0,5 und 90%.

Die Anwendung der erfindungsgemässen Wirkstoffkombinationen erfolgt in Form ihrer handelsüblichen Formulierungen und/oder den aus diesen Formulierungen bereiteten Anwendungsformen.

Der gesamte Wirkstoffgehalt (einschliesslich Synergist) der aus den handelsüblichen Formulierungen bereiteten Anwendungsformen kann in weiten Bereichen variieren. Die Wirkstoffkonzentration der Anwendungsformen kann von 0,0001 bis zu 100 Gew.-% Wirkstoffkombination, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Anhand der folgenden Beispiele soll die Wirksamkeit der erfindungsgemäss verwendbaren Verbindungen der Formel (I) erläutert werden:

I. *Beispiele für erfindungsgemäss verwendbare Wirkstoffe*

| Kennbuchstabe | Formel | Name |
|---|---|---|
| A) | 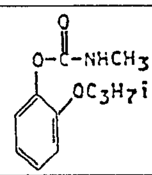 | (Propoxur) |
| B) | 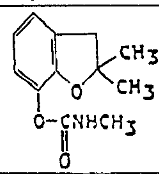 | (Carbofuran) |
| C) | 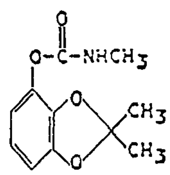 | (Bendiocarb) |
| D) | 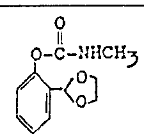 | (Dioxacarb) |
| E) | $CH_3{-}HN{-}\overset{\overset{O}{\|}}{C}{-}O{-}N{=}\underset{\underset{CH_3}{\mid}}{C}{-}S{-}CH_3$ | (Methomyl) |
| F) | Pyrethrine natürlicher Herkunft als 25%iger Extrakt | |
| G) | 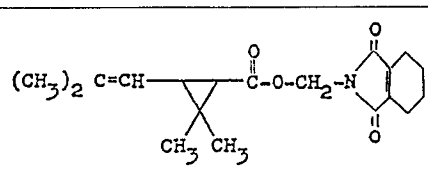 | (Tetramethrin) |
| H) | 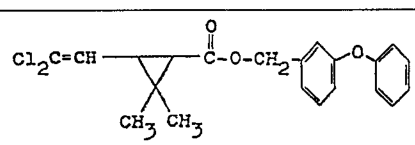 | (Permethrin) |
| I) | 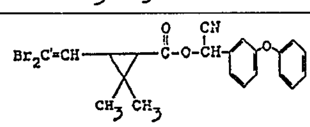 | (Decamethrin) |

| Kenn-buch-stabe | Formel | Name |
|---|---|---|
| J) | 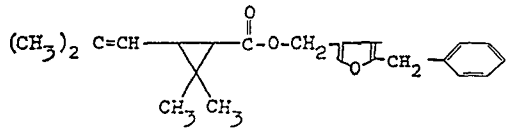 | (Resmethrin) |
| K) | 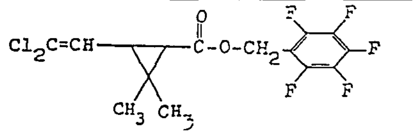 | |
| L) | 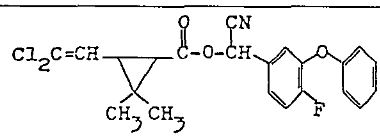 | |
| M) | 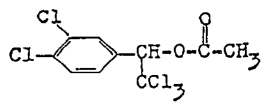 | (Penfenate) |
| N) | $Cl_2C{=}CH{-}O{-}\overset{O}{\overset{\Vert}{P}}\,(OCH_3)_2$ | (DDVP) |
| O) | $(CH_3O)_2\,\overset{O}{\overset{\Vert}{P}}{-}S{-}CH_2{-}\overset{O}{\overset{\Vert}{C}}{-}N\begin{smallmatrix}\diagup CH_3\\ \diagdown H\end{smallmatrix}$ | (Omethoat) |

II. *Beispiele von erfindungsgemäss verwendbaren Synergisten*

| Nr. | Formel |
|---|---|
| 1. | 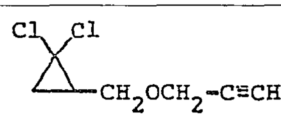 |
| 2. | 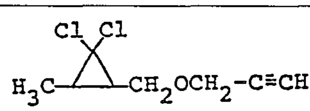 |
| 3. | 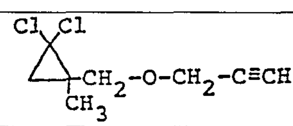 |
| 4. | 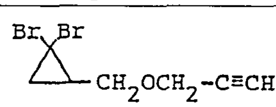 |
| 5. | 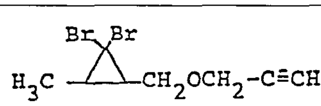 |
| 6. | 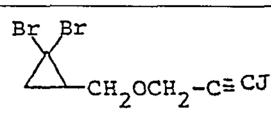 |
| 7. | 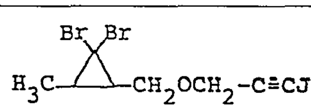 |

| Nr. | Formel |
|---|---|
| 8. | 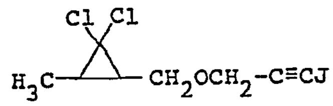 |
| 9. | 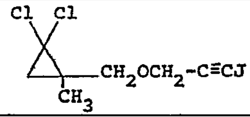 |
| 10. | 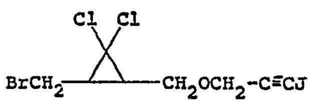 |
| 11. | 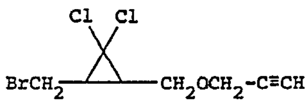 |
| 12. | 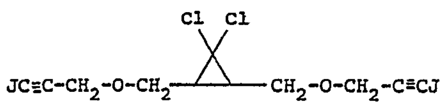 |
| 13. | 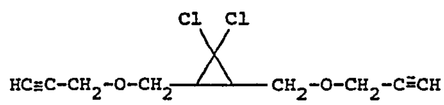 |
| 14. | 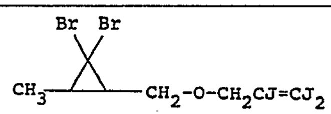 |
| Vergleichsverbindung: Piperonylbutoxid (bekannt) | |
| 15. | 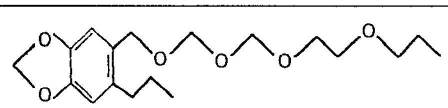 |

III. *Testdurchführung*

$LT_{100}$-Test
Testtiere: Gegen Phosphorsäureester und Carbamate resistente weibliche *Musca domestica* (Stamm Weymanns)
Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipettiert. Das Filterpapier saugt die Lösungen auf. Die Petrischalen bleiben so lange offen stehen, bis das Lösungsmittel vollständig verdunstet ist. Anschliessend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100%ige knock-down-Wirkung erforderlich ist. Wird die $LT_{100}$ nach 6 Stunden nicht erreicht, so wird der Prozentsatz der knock-down gegangenen Testtiere festgestellt.

Konzentrationen der Wirkstoffe, Synergisten und Gemische und ihre Wirkungen gehen aus der nachfolgenden Tabelle hervor. Die Versuche wurden in 2 Versuchsreihen (1 und 2) in direkten Vergleichsversuchen durchgeführt. Daher ergaben sich gewisse Wirkungsunterschiede bei einigen Wirkstoffen (ohne Synergistenbeimischung).

IV. *Testergebnisse*

$Lt_{100}$-Test mit gegen Phosphorsäureester und Carbamate resistenten weiblichen *Musca domestica* (Stamm Weymanns).

*Versuchsreihe 1:*

| Wirkstoffe/Synergist | Konzentration (%) Wirkstoff/Synergist | $Lt_{100}$ in oder bei 360′ in % |
|---|---|---|
| A | 1,0 | 360′=20% |
| B | 1,0 | 360′=25% |

| Wirkstoffe/Synergist | Konzentration (%) Wirkstoff/Synergist | $Lt_{100}$ in oder bei 360′ in % |
|---|---|---|
| C | 1,0 | 360′= 0% |
| E | 0,2 | 360′=95% |
| F | 0,2 | 150′ |
| G | 0,2 | 180′ |
| H | 0,04 | 360′ |
| I | 0,008 | 90′ |
| J | 0,04 | 240′ |
| M | 1,0 | 360′=80% |
| N | 0,008 | 360′=95% |
| 1) | 1,0 | 360′=40% |
| 2) | 1,0 | 360′= 0% |
| 3) | 1,0 | 360′= 5% |
| 4) | 1,0 | 360′=40% |
| 5) | 1,0 | 360′=50% |
| 15) (bekannt) | 1,0 | 360′= 0% |
| A +15 | 1,0 +1,0 | 240′ |
| A + 1 | 0,04 +0,04 | 150′ |
| A + 2 | 0,04 +0,04 | 90′ |
| A + 3 | 0,04 +0,04 | 105′ |
| A + 4 | 0,04 +0,04 | 90′ |
| A + 5 | 0,04 +0,04 | 105′ |
| B +15 | 0,04 +0,04 | 360′=50% |
| B + 1 | 0,04 +0,04 | 210′ |
| B + 2 | 0,04 +0,04 | 180′ |
| B + 4 | 0,04 +0,04 | 180′ |
| B + 5 | 0,04 +0,04 | 120′ |
| C +15 | 0,2 +0,2 | 360′=80% |
| C + 1 | 0,04 +0,04 | 150′ |
| C + 2 | 0,04 +0,04 | 150′ |
| C + 3 | 0,04 +0,04 | 150′ |
| C + 4 | 0,04 +0,04 | 180′ |
| C + 5 | 0,04 +0,04 | 150′ |
| E +15 | 0,04 +0,04 | 360′=30% |
| E + 1 | 0,04 +0,04 | 150′ |
| E + 2 | 0,04 +0,04 | 180′ |
| E + 3 | 0,04 +0,04 | 105′ |
| E + 4 | 0,04 +0,04 | 150′ |
| E + 5 | 0,04 +0,04 | 150′ |
| F + 2 | 0,2 +0,2 | 75′ |
| F + 4 | 0,2 +0,2 | 75′ |
| F + 5 | 0,2 +0,2 | 75′ |
| G + 1 | 0,2 +0,2 | 120′ |
| G + 2 | 0,2 +0,2 | 105′ |
| G + 4 | 0,2 +0,2 | 90′ |
| G + 5 | 0,2 +0,2 | 60′ |

| Wirkstoffe/Synergist | Konzentration (%) Wirkstoff/Synergist | $Lt_{100}$ in oder bei 360' in % |
|---|---|---|
| H +15 | 0,04 +0,04 | 120' |
| H + 1 | 0,04 +0,04 | 105' |
| H + 2 | 0,04 +0,04 | 90' |
| H + 4 | 0,04 +0,04 | 60' |
| H + 5 | 0,04 +0,04 | 105' |
| I + 1 | 0,008 +0,008 | 60' |
| I + 2 | 0,008 +0,008 | 60' |
| I + 4 | 0,008 +0,008 | 60' |
| I + 5 | 0,008 +0,008 | 60' |
| J + 1 | 0,04 +0,04 | 150' |
| J + 2 | 0,04 +0,04 | 150' |
| J + 4 | 0,04 +0,04 | 120' |
| J + 5 | 0,04 +0,04 | 105' |
| M +15 | 1,0 +1,0 | 360'=95% |
| M + 1 | 1,0 +1,0 | 210' |
| M + 2 | 0,2 +0,2 | 210' |
| M + 3 | 0,2 +0,2 | 210' |
| M + 4 | 0,2 +0,2 | 150' |
| M + 5 | 0,2 +0,2 | 180' |
| N +15 | 0,008 +0,008 | 90' |
| N + 1 | 0,008 +0,008 | 75' |
| N + 2 | 0,008 +0,008 | 90' |
| N + 3 | 0,008 +0,008 | 75' |
| N + 4 | 0,008 +0,008 | 75' |
| N + 5 | 0,008 +0,008 | 75' |

*Versuchsreihe 2:*

| Wirkstoffe/Synergist | Konzentration (%) Wirkstoff/Synergist | $Lt_{100}$ in oder bei 360' in % |
|---|---|---|
| A) | 1,0 | 360'= 5% |
| B) | 0,2 | 360'=40% |
| C) | 1,0 | 360'= 0% |
| D) | 1,0 | 360'= 0% |
| E) | 0,2 | 360'=30% |
| F) | 0,04 | 360'=20% |
| G) | 0,04 | 360'=50% |
| H) | 0,008 | 210' |
| L) | 0,0016 | 360'=95% |
| M) | 0,04 | 360'=30% |
| N) | 0,04 | 90' |
| O) | 0,04 | 360'=10% |

| Wirkstoffe/Synergist | | Konzentration (%) Wirkstoff/Synergist | | $Lt_{100}$ in oder bei 360′ in % |
|---|---|---|---|---|
| | 6) | | 1,0 | 360′= 0% |
| | 7) | | 1,0 | 360′=30% |
| | 8) | | 0,2 | 360′=40% |
| | 9) | | 0,2 | 360′=80% |
| | 10) | | 1,0 | 360′= 0% |
| | 11) | | 0,2 | 360′= 0% |
| | 12) | | 1,0 | 360′= 0% |
| | 13) | | 1,0 | 360′=30% |
| | 14) | | 1,0 | 360′= 0% |
| | 15) (bekannt) | | 1,0 | 360′= 0% |
| A | + 6 | 0,2 | +0,2 | 105′ |
| A | + 7 | 0,04 | +0,04 | 240′ |
| A | + 8 | 0,04 | +0,04 | 150′ |
| A | + 9 | 0,04 | +0,04 | 120′ |
| A | +10 | 0,2 | +0,2 | 360′ |
| A | +11 | 0,2 | +0,2 | 120′ |
| A | +12 | 0,2 | +0,2 | 360′=90% |
| A | +13 | 0,04 | +0,04 | 210′ |
| A | +15 | 0,2 | +0,2 | 360′=45% |
| B | + 6 | 0,04 | +0,04 | 150′ |
| B | + 7 | 0,2 | +0,2 | 150′ |
| B | + 8 | 0,04 | +0,04 | 150′ |
| B | + 9 | 0,04 | +0,04 | 105′ |
| B | +10 | 0,2 | +0,2 | 360′=80% |
| B | +11 | 0,04 | +0,04 | 180′ |
| B | +12 | 0,2 | +0,2 | 360′ |
| B | +13 | 0,04 | +0,04 | 180′ |
| B | +15 | 0,2 | +0,2 | 360′=40% |
| C | + 6 | 0,2 | +0,2 | 150′ |
| C | + 7 | 0,2 | +0,2 | 360′ |
| C | + 8 | 0,2 | +0,2 | 120′ |
| C | + 9 | 0,04 | +0,04 | 150′ |
| C | +11 | 0,2 | +0,2 | 240′ |
| C | +13 | 0,2 | +0,2 | 150′ |
| C | +15 | 0,2 | +0,2 | 360′=40% |
| D | + 6 | 0,2 | +0,2 | 240′ |
| D | + 7 | 0,2 | +0,2 | 360′=90% |
| D | + 8 | 0,2 | +0,2 | 240′ |
| D | + 9 | 0,2 | +0,2 | 210′ |
| D | +11 | 0,2 | +0,2 | 360′ |
| D | +13 | 0,2 | +0,2 | 360′ |
| D | +15 | 0,2 | +0,2 | 360′=20% |

| Wirkstoffe/Synergist | | Konzentration (%) Wirkstoff/Synergist | | $Lt_{100}$ in oder bei 360′ in % |
|---|---|---|---|---|
| E | + 6 | 0,2 | +0,2 | 180′ |
| E | + 7 | 0,2 | +0,2 | 180′ |
| E | + 8 | 0,2 | +0,2 | 150′ |
| E | + 9 | 0,2 | +0,2 | 105′ |
| E | +10 | 0,2 | +0,2 | 360′=90% |
| E | +11 | 0,2 | +0,2 | 120′ |
| E | +12 | 0,2 | +0,2 | 210′ |
| E | +13 | 0,2 | +0,2 | 150′ |
| E | +14 | 0,2 | +0,2 | 210′ |
| F | + 6 | 0,04 | +0,04 | 180′ |
| F | + 7 | 0,04 | +0,04 | 240′ |
| F | + 8 | 0,04 | +0,04 | 180′ |
| F | + 9 | 0,04 | +0,04 | 180′ |
| F | +11 | 0,04 | +0,04 | 180′ |
| F | +13 | 0,04 | +0,04 | 150′ |
| G | + 6 | 0,04 | +0,04 | 90′ |
| G | + 7 | 0,04 | +0,04 | 210′ |
| G | + 8 | 0,04 | +0,04 | 90′ |
| G | + 9 | 0,04 | +0,04 | 90′ |
| G | +10 | 0,04 | +0,04 | 210′ |
| G | +11 | 0,04 | +0,04 | 105′ |
| G | +12 | 0,04 | +0,04 | 180′ |
| G | +13 | 0,04 | +0,04 | 90′ |
| G | +14 | 0,04 | +0,04 | 210′ |
| H | + 6 | 0,008 | +0,008 | 90′ |
| H | + 8 | 0,008 | +0,008 | 105′ |
| H | + 9 | 0,008 | +0,008 | 105′ |
| H | +13 | 0,008 | +0,008 | 105′ |
| H | +15 | 0,008 | +0,008 | 150′ |
| L | + 6 | 0,0016 | +0,0016 | 120′ |
| L | + 7 | 0,0016 | +0,0016 | 120′ |
| L | + 8 | 0,0016 | +0,0016 | 120′ |
| L | + 9 | 0,0016 | +0,0016 | 150′ |
| L | +10 | 0,0016 | +0,0016 | 210′ |
| L | +11 | 0,0016 | +0,0016 | 120′ |
| L | +12 | 0,0016 | +0,0016 | 105′ |
| L | +13 | 0,0016 | +0,0016 | 105′ |
| L | +14 | 0,0016 | +0,0016 | 105′ |
| M | + 6 | 0,04 | +0,04 | 240′ |
| M | + 8 | 0,04 | +0,04 | 210′ |
| M | + 9 | 0,04 | +0,04 | 240′ |
| M | +15 | 0,04 | +0,04 | 360′=80% |
| N | + 7 | 0,04 | +0,04 | 75′ |
| N | + 9 | 0,04 | +0,04 | 45′ |

| Wirkstoffe/Synergist | | Konzentration (%) Wirkstoff/Synergist | | $Lt_{100}$ in oder bei 360' in % |
|---|---|---|---|---|
| N | +10 | 0,04 | +0,04 | 60' |
| N | +11 | 0,04 | +0,04 | 60' |
| N | +12 | 0,04 | +0,04 | 75' |
| N | +14 | 0,04 | +0,04 | 75' |
| O | + 6 | 0,04 | +0,04 | 360' |
| O | + 7 | 0,04 | +0,04 | 360' |
| O | + 8 | 0,04 | +0,04 | 240' |
| O | + 9 | 0,04 | +0,04 | 150' |
| O | +10 | 0,04 | +0,04 | 360' |
| O | +11 | 0,04 | +0,04 | 360' |
| O | +13 | 0,04 | +0,04 | 240' |

Die Herstellung der Verbindungen der Formel (I) sei anhand der folgenden Herstellungsbeispiele erläutert (alle Prozentangaben beziehen sich – wo nichts anderes angegeben wird – auf Gewichtsprozente).

*Beispiel 1:*

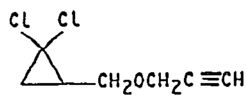

(Verfahrensvariante a) Synergist Nr. 1

Zu 100 ml auf 0°C gekühltem Propargylalkohol werden in kleinen Portionen 3 g Natriumhydrid (80% in Paraffinöl) gegeben; während der Zugabe wird die Temperatur auf 0 bis 5°C gehalten. Man rührt weitere 10 Minuten bei dieser Temperatur und tropft anschliessend 21 g (0,1 Mol) 1,1-Dichlor-2-brommethylcyclopropan innerhalb von 10 Minuten bei 0°C hinzu. Man erhitzt 14 Stunden auf 70°C und kühlt auf etwa 20°C ab. Nach Absaugen des festen Rückstandes wird mit 200 ml Methylenchlorid nachgewaschen. Die organische Phase wird im Wasserstrahlvakuum bei 80°C Badtemperatur vom Lösungsmittel und vom überschüssigen Propargylalkohol befreit. Der Rückstand wird in 200 ml Methylenchlorid aufgenommen und mit 50 ml Wasser gewaschen. Die wässerige Phase wird nochmals mit 100 ml Methylenchlorid extrahiert. Die vereinigten organischen Phasen werden eingedampft und anschliessend rektifiziert.

Man erhält 7,8 g (43% der Theorie) 3-(2,2-Dichlorcyclopropylmethoxy)-1-propin vom Brechungsindex $n_D^{20}$:1,4850.

*Beispiel 2:*

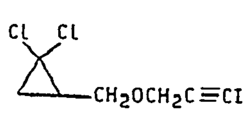

(Verfahrensvariante b)

4,5 g (0,025 Mol) 3-(2,2-Dichlorcyclopropylmethoxy)-1-propin werden in 100 ml Methanol gelöst und bei 20°C portionsweise und in stetigem Wechsel mit 6,3 g (0,025 Mol) Iod und 2,5 g 45%iger Natronlauge versetzt. Nach der Zugabe wird noch 1 Stunde bei 20°C gerührt. Nach Abdestillieren des Methanols wird der Rückstand in 100 ml Dichlormethan aufgenommen und mit 50 ml verdünnter Natriumthiosulfatlösung und anschliessend mit 50 ml Wasser gewaschen. Die organische Phase wird über Magnesiumsulfat getrocknet.

Man erhält nach Andestillieren bei 1 mbar (100 Pa) und 95°C 3,8 g (50% der Theorie) 3-(2,2-Dichlorcyclopropylmethoxy)-1-iod-1-propion vom Brechungsindex $n_D^{20}$:1,5818.

*Beispiel 3:*

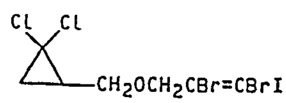

(Verfahrensvariante c)

3 g (0,01 Mol) 3-(2,2-Dichlorcyclopropylmethoxy)-1-iod-1-propin werden in 50 ml Methylenchlorid gelöst und bei 20°C mit 2 g Brom (0,0125 Mol) in 20 ml Methylenchlorid versetzt. Anschliessend wird 14 Stunden bei 60°C gerührt. Nach Abkühlung auf 20°C wird das Gemisch mit wässeriger 20 ml Natriumthiosulfatlösung und dann mit Wasser gewaschen. Die organische Phase wird über Magnesiumsulfat getrocknet und das Lösungsmittel im Wasserstrahlvakuum entfernt.

Man erhält 4,2 g (90% der Theorie) 1,2-Dibrom-3-(2,2-dichlorcyclopropylmethoxy)-1-iod-1-propen vom Brechungsindex $n_D^{20}$:1,6014.

*Beispiel 4:*

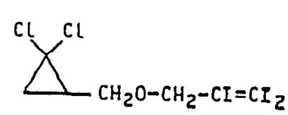

(Verfahrensvariante b)

23 g (0,128 Mol) 3-(2,2-Dichlorcyclopropyl-

methoxy)-1-propin in 100 ml Methanol werden bei 20°C unter Rühren portionsweise und im stetigen Wechsel mit 96 g (0,378 Mol) Iod und 13 g 45%iger Natronlauge versetzt und 14 Stunden gerührt. Das Lösungsmittel wird anschliessend im Wasserstrahlvakuum abdestilliert und der Rückstand mit 500 ml Methylenchlorid versetzt. Bis zur Entfärbung wird zunächst mit 200 ml verdünnter Natriumthiosulfatlösung und dann mit 200 ml Wasser gewaschen. Die organische Phase wird über Magnesiumsulfat getrocknet und im Wasserstrahlvakuum eingeengt.

Man erhält 46 g (64% der Theorie) 3-(2,2-Dichlorcyclopropylmethoxy)-1,1,2-triiod-1-propin vom Brechungsindex $n_D^{20}$:1,6623.

Analog Verfahrensvariante (a), (b), oder (c) können die in der nachstehenden Tabelle 4 aufgeführten Verbindungen der Formel (I) hergestellt werden:

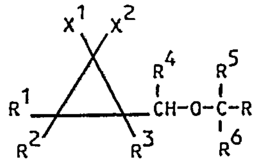
(I)

*(Tabelle auf der nächsten Seite)*

Die Herstellung von Verbindungen der Formel (II) sei anhand der folgenden Beispiele erläutert:

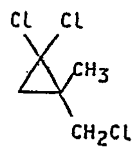

Zu einem Gemisch aus 90,5 g (1 Mol) 3-Chlor-2-methyl-1-propen, 1,5 l Chloroform, 6 g Lithiumbromid, 2,8 g Triethylbenzylammoniumbromid und 24 ml Ethanol werden unter kräftigem Rühren innerhalb von 2 Stunden 1440 ml einer frisch bereiteten 50%igen Natronlauge getropft. Während des Zutropfens steigt die Temperatur im Reaktionsgefäss auf 57 bis 58°C an und wird während der ganzen Reaktionszeit konstant gehalten. Nach Beendigung der Zugabe wird die Lösung noch 1 Stunde kräftig gerührt und dann auf Raumtemperatur abgekühlt. Nach Zugabe von 1 l Chloroform und 1 l Wasser wird die organische Phase mehrmals mit Wasser gewaschen und anschliessend über Magnesiumsulfat getrocknet. Nach Entfernen des Lösungsmittels im Wasserstrahlvakuum und anschliessender Rektifikation erhält man 127 g (73% der Theorie) 1-Chlormethyl-2,2-dichlor-2-methylcyclopropan vom Brechungsindex $n_D^{20}$:1,4860.

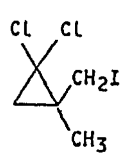

Eine Mischung von 300 ml Aceton, 82,5 g (0,55 Mol) Natriumiodid und 86,5 g (0,55 Mol) 1-Chlormethyl-2,2-dichlor-2-methylcyclopropan wird 48 Stunden bei 62°C erhitzt. Nach Abkühlung auf Raumtemperatur wird das Reaktionsprodukt im Wasserstrahlvakuum bei 50°C eingeengt. Der Rückstand wird in 1 l Methylenchlorid aufgenommen und mit 300 ml Wasser, 300 ml verdünnter Natriumthiosulfatlösung und erneut mit 300 ml Wasser gewaschen. Die organische Phase wird über Magnesiumsulfat getrocknet und eingeengt.

Nach Destillation über eine 20-cm-Vigreux-Kolonne erhält man 121 g (83% der Theorie) 1,1-Dichlor-2-iodmethyl-2-methylcyclopropan vom Brechungsindex $n_D^{20}$:1,5261.

Die übrigen Verbindungen der Formel (II) können auf analoge Weise erhalten werden.

## Patentansprüche

1. Substituierte Cyclopropylmethyl(en)ether der Formel (I)

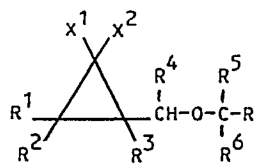
(I)

in welcher

$X^1$ und $X^2$ gleich oder verschieden sind und für Fluor, Chlor und/oder Brom stehen,

$R^1$ für Wasserstoff oder $C_1$-$C_6$-Alkyl steht,

$R^2$ und $R^3$ gleich oder verschieden sind und für Wasserstoff, $C_1$-$C_6$-Alkyl, $C_1$-$C_4$-Halogenalkyl, 1-Pyrazolylmethyl, 1-Imidazolylmethyl, 1-(1,2,4-Triazolyl)methyl, $C_1$-$C_4$-Alkoxy-$C_1$-$C_4$-alkyl, $C_1$-$C_4$-Alkylthio-$C_1$-$C_4$-alkyl, (Di)$C_1$-$C_4$-Alkylamino-$C_1$-$C_4$-alkyl oder für gegebenenfalls durch Halogen, wie insbesondere Chlor, Brom oder Iod, substituierte Reste der Reihe Phenyl, $C_2$-$C_4$-Alkenoxy-$C_1$-$C_2$-alkyl und $C_2$-$C_4$-Alkinoxy-$C_1$-$C_2$-alkyl stehen,

$R^4$ für Wasserstoff oder $C_1$-$C_4$-alkyl steht,

$R^5$ und $R^6$ gleich oder verschieden sind und für Wasserstoff, $C_1$-$C_4$-Alkyl oder für gegebenenfalls durch Halogen, wie insbesondere Chlor, Brom oder Iod substituierte Reste der Reihe Phenyl, Benzyl und Phenylethyl stehen, und

R für Triiod-$C_2$-$C_4$-alkenyl, Dibromiod-$C_2$-$C_4$-alkenyl, Dichloriod-$C_2$-$C_4$-alkenyl, $C_2$-$C_4$-Alkinyl oder Iod-$C_2$-$C_4$-alkinyl steht, wobei sich die Mehrfachbindungen jeweils am Ende der Kette befinden und im Falle von Trihalogenalkenyl ein Iodatom an das letzte Kohlenstoffatom und die beiden übrigen Halogenatome an die beiden letzten Kohlenstoffatome der Kette gebunden sind und im Falle von Iodalkinyl das Iodatom an das letzte Kohlenstoffatom der Kette gebunden ist.

2. Substituierte Cyclopropylmethyl(en)ether gemäss Formel (I) nach Anspruch 1, in welcher

$X^1$ und $X^2$ gleich oder verschieden sind und für Chlor und/oder Brom stehen,

*Tabelle 4:*

| Beispiel Nr. | $X^1$ | $X^2$ | $R^1$ | $R^2$ | $R^3$ | $R^4$ | $R^5$ | $R^6$ | R | Synergist Nr. | Brechungsindex $n_D^{20}$ |
|---|---|---|---|---|---|---|---|---|---|---|---|
| 5 | Br | Br | H | $CH_3$ | H | H | H | H | $-C\equiv CH$ | 5 | 1,5300 |
| 6 | Cl | Cl | H | $CH_3$ | H | H | H | H | $-C\equiv CH$ | 2 | 1,4826 |
| 7 | Br | Br | H | H | H | H | H | H | $-C\equiv CH$ | 4 | 1,5358 |
| 8 | Br | Br | H | H | H | H | H | H | $-C\equiv CI$ | 6 | 1,5980 |
| 9 | Cl | Cl | H | $CH_3$ | H | H | H | H | $-C\equiv CI$ | 8 | 1,5540 |
| 10 | Cl | Cl | H | H | $CH_3$ | H | H | H | $-C\equiv CI$ | 9 | 1,5530 |
| 11 | Br | Br | H | $CH_3$ | H | H | H | H | $-C\equiv CI$ | 7 | 1,5620 |
| 12 | Cl | Cl | H | H | $CH_3$ | H | H | H | $-C\equiv CH$ | 3 | 1,4952 |
| 13 | Cl | Cl | H | $CH_3$ | H | H | H | H | $-C=CI$<br>\| \|<br>Cl Cl | | 1,5484 |
| 14 | Br | Br | H | $CH_3$ | H | H | H | H | $-C=CI$<br>\| \|<br>Cl Cl | | 1,5780 |
| 15 | Br | Br | H | $CH_3$ | H | H | H | H | $-CI=CI_2$ | 14 | 1,6496 |
| 16 | Cl | Cl | H | $CH_3$ | H | H | H | H | $-CBr=CBrI$ | | 1,5920 |
| 17 | Cl | Cl | H | $CH_3$ | H | H | H | H | $-CI=CI_2$ | | 1,6396 |
| 18 | Cl | Cl | H | $-CH_2Br$ | H | H | H | H | $-C\equiv CH$ | 11 | 1,5125 |
| 19 | Cl | Cl | H | $-CH_2OCH_2C\equiv CH$ | H | H | H | H | $-C\equiv CH$ | 13 | 1,5075 |
| 20 | Cl | Cl | H | $-CH_2Br$ | H | H | H | H | $-C\equiv CI$ | 10 | 1,5565 |
| 21 | Cl | Cl | H | $-CH_2OCH_2C\equiv CI$ | H | H | H | H | $-C\equiv CI$ | 12 | 1,5920 |
| 22 | Cl | Cl | H | H | $CH_3$ | H | H | H | $-CBr=CBrI$ | | 1,5391 |
| 23 | Cl | Cl | H | H | $CH_3$ | H | H | H | $-CI=CI_2$ | | 1,6464 |
| 24 | Cl | Cl | H | H | $-CH_2Cl$ | H | H | H | $-C\equiv CH$ | | 1,4962 |
| 25 | Cl | Cl | H | H | $CH_3$ | H | H | H | $-C=CI$<br>\| \|<br>Cl Cl | | 1,5547 |

$R^1$ für Wasserstoff, Methyl, Ethyl, n-Propyl oder n-Butyl steht,

$R^2$ und $R^3$ gleich oder verschieden sind und für Wasserstoff, Methyl, Ethyl, h-Propyl, n-Butyl, 1-Pyrazolylmethyl, 1-Imidazolylmethyl, 1-(1,2,4-Triazolyl)methyl, Methoxymethyl, Ethoxymethyl, Ethoxyethyl, Methylthiomethyl, Ethylthiomethyl, Ethylthioethyl, (Di)Methylaminomethyl, (Di)-Methylaminoethyl, (Di)Ethylaminomethyl, (Di)-Ethylaminoethyl, Propenyloxymethyl, Propinyloxymethyl, Iodpropinyloxymethyl, Triiodpropenyloxymethyl, Dichloriodpropenyloxymethyl, Dibromiodpropenyloxymethyl, Phenyl, 2-, 3-, 4-Chlorphenyl, 2,4-Dichlorphenyl, 2-, 3-, 4-Bromphenyl, 2,4-Dibromphenyl, Chlormethyl und Brommethyl stehen,

$R^4$ für Wasserstoff, Methyl, Ethyl, n- oder i-Propyl steht,

$R^5$ und $R^6$ gleich oder verschieden sind und für Wasserstoff, Methyl, Ethyl, n-Propyl, i-Propyl, 2-, 3-, 4-Chlorphenyl, 2,4-Dichlorphenyl oder 4-Bromphenyl stehen, und

R für Triiodethenyl, Dichloriodethenyl, Dibromiodethenyl, Ethinyl, Iodethinyl, Propinyl oder Iodpropinyl steht, wobei in den Iod enthaltenden Resten ein Iodatom jeweils an das letzte Kohlenstoffatom der Kette gebunden ist.

3. Substituierte Cyclopropylmethyl(en)ether gemäss Formel (I) nach Anspruch 1, in welcher

$X^1$ und $X^2$ gleich oder verschieden sind und für Chlor und/oder Brom stehen,

$R^1$ für Wasserstoff, Methyl, oder Ethyl steht,

$R^2$ und $R^3$ gleich oder verschieden sind und für Wasserstoff, Methyl, Ethyl, Chlormethyl, Brommethyl, 1-Propenyloxymethyl, 1-Propinyloxymethyl oder 1-Iodpropinyloxymethyl stehen,

$R^4$ für Wasserstoff oder Methyl steht,

$R^5$ und $R^6$ gleich oder veschieden sind und für Wasserstoff oder Methyl stehen, und

R für Triiodethenyl, Dichloriodethenyl, Dibromiodethenyl, Ethinyl oder Iodethinyl steht, wobei in den Iod enthaltenden Resten ein Iodatom jeweils an das letzte Kohlenstoffatom der Kette gebunden ist.

4. Verfahren zur Herstellung von substituierten Cyclopropylmethyl(en)ethern der allgemeinen Formel (I)

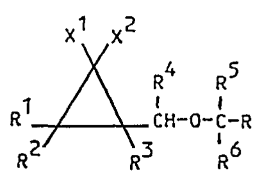 (I)

in welcher

$X^1$ und $X^2$ gleich oder verschieden sind und für Fluor, Chlor und/oder Brom stehen,

$R^1$ für Wasserstoff oder $C_1$-$C_6$-Alkyl steht,

$R^2$ und $R^3$ gleich oder verschieden sind und für Wasserstoff, $C_1$-$C_6$-Alkyl, $C_1$-$C_4$-Halogenalkyl, 1-Pyrazolylmethyl, 1-Imidazolylmethyl, 1-(1,2,4-Triazolyl)methyl, $C_1$-$C_4$-Alkoxy-$C_1$-$C_4$-alkyl, $C_1$-$C_4$-Alkylthio-$C_1$-$C_4$-alkyl, (Di)$C_1$-$C_4$-Alkylamino-$C_1$-$C_4$-alkyl oder für gegebenenfalls durch Halogen, wie insbesondere Chlor, Brom oder Iod, substituierte Reste der Reihe Phenyl, $C_2$-$C_4$-Alkenoxy-$C_1$-$C_2$-alkyl und $C_2$-$C_4$-Alkinoxy-$C_1$-$C_2$-alkyl stehen,

$R^4$ für Wasserstoff oder $C_1$-$C_4$-Alkyl steht,

$R^5$ und $R^6$ gleich oder verschieden sind und für Wasserstoff, $C_1$-$C_4$-Alkyl oder für gegebenenfalls durch Halogen, wie insbesondere Chlor, Brom oder Iod substituierte Reste der Reihe Phenyl, Benzyl und Phenylethyl stehen, und

R für Triiod-$C_2$-$C_4$-alkenyl, Dibromiod-$C_2$-$C_4$-alkenyl, Dichloriod-$C_2$-$C_4$-alkenyl, $C_2$-$C_4$-Alkinyl oder Iod-$C_2$-$C_4$-alkinyl steht, wobei sich die Mehrfachbindungen jeweils am Ende der Kette befinden und im Falle von Trihalogenalkenyl ein Iodatom an das letzte Kohlenstoffatom und die beiden übrigen Halogenatome an die beiden letzten Kohlenstoffatome der Kette gebunden sind und im Falle von Iodalkinyl das Iodatom an das letzte Kohlenstoffatom der Kette gebunden ist, dadurch gekennzeichnet, dass man

a) Cyclopropylmethyl(en)halogenide der allgemeinen Formel (II)

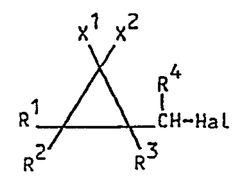 (II)

in welcher

$R^1$, $R^2$, $R^3$, $R^4$, $X^1$ und $X^2$ die oben angegebene Bedeutung haben, und

Hal für Halogen, wie insbesondere Chlor, Brom oder Iod steht, mit Alkoholen der allgemeinen Formel (III)

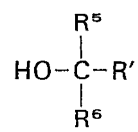 (III)

in welcher

$R^5$ und $R^6$ die oben angegebene Bedeutung haben, und

R' für $C_2$-$C_4$-Alkinyl steht, wobei sich die Mehrfachbindung am Ende der Kette befindet,

oder deren Salze, gegebenenfalls in Gegenwart von Säureakzeptoren und/oder gegebenenfalls in Gegenwart von Verdünnungsmitteln, umsetzt, oder

b) die nach Verfahrensvariante (a) hergestellten neuen Verbindungen der allgemeinen Formel (Ia)

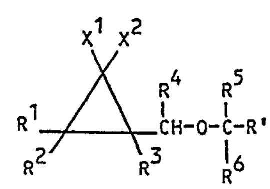 (Ia)

in welcher

$R^1$, $R^2$, $R^3$, $R^4$, $R^5$, $R^6$, R', $X^1$ und $X^2$ die oben angegebenen Bedeutungen haben, mit 0,5 bis 6 Mol Iod in Gegenwart von Säureakzeptoren und

gegebenenfalls in Gegenwart eines Verdünnungsmittels umsetzt, oder

c) die nach Verfahren (b) hergestellten neuen Verbindungen der allgemeinen Formel (Ib)

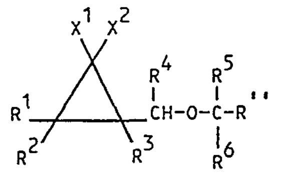
(Ib)

in welcher

$R^1$, $R^2$, $R^3$, $R^4$, $R^5$, $R^6$, $X^1$ und $X^2$ die oben angegebenen Bedeutungen haben, und R'' für Iod-$C_2$-$C_4$-alkinyl steht, wobei sich die Mehrfachbindung am Ende der Kette befindet und das Iodatom an das letzte Kohlenstoffatom gebunden ist, mit Chlor, Brom oder Iod, gegebenenfalls in Gegenwart eines Verdünnungsmittels, umsetzt.

5. Schädlingsbekämpfungsmittel, gekennzeichnet durch einen Gehalt an mindestens einer Verbindung der Formel (I) gemäss Anspruch 1 oder 4.

6. Schädlingsbekämpfungsmittel enthaltend wenigstens einen Synergisten und wenigstens einen gegen Arthropoden wirksamen anderen Stoff, gekennzeichnet durch einen Gehalt an mindestens einer Verbindung der Formel (I) gemäss Anspruch 1 oder 4 als Synergist.

7. Verwendung von Verbindungen der Formel (I) gemäss Anspruch 1 oder 4 als Synergisten bei der Bekämpfung von Schädlingen, insbesondere Insekten und Spinnentieren.

8. Verfahren zur Bekämpfung von Schädlingen, dadurch gekennzeichnet, dass man Verbindungen der Formel (I) gemäss Anspruch 1 oder 4 und andere Wirkstoffe, welche gegen Arthropoden wirksam sind, auf die Schädlinge, vorzugsweise Insekten oder Spinnentiere, oder ihren Lebensraum einwirken lässt.

9. Verfahren zur Herstellung von Schädlingsbekämpfungsmitteln, dadurch gekennzeichnet, dass man Verbindungen der Formel (I) gemäss Anspruch 1 oder 4 mit anderen Wirkstoffen, welche gegen Arthropoden wirksam sind, und mit Streckmitteln und/oder oberflächenaktiven Mitteln vermischt.

## Revendications

1. Ethers-oxydes de cyclopropylméthyle(ène) substitués, de formule (I)

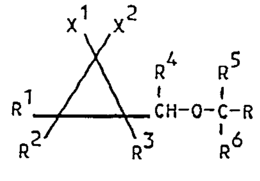
(I)

dans laquelle

$X^1$ et $X^2$ sont identiques ou différents et représentent un atome de fluor, de chlore et/ou de brome,

$R^1$ représente un atome d'hydrogène ou un reste alkyle en $C_1$-$C_6$,

$R^2$ et $R^3$ sont identiques ou différents et représentent un atome d'hydrogène, un reste alkyle en $C_1$-$C_6$, halogénoalkyle en $C_1$-$C_4$, 1-pyrazolylméthyle, 1-imidazolylméthyle, 1-(1,2,4-triazolyl)-méthyle, alcoxy(en $C_1$-$C_4$)-alkyle en $C_1$-$C_4$, alkyl(en $C_1$-$C_4$)-thioalkyle en $C_1$-$C_4$, (di)(alkyl en $C_1$-$C_4$)-aminoalkyle en $C_1$-$C_4$ ou des restes (éventuellement substitués par de l'halogène, comme en particulier le chlore, le brome ou l'iode) de l'ensemble formé par un reste phényle, alcénoxy(en $C_1$-$C_4$)-alkyle en $C_1$-$C_2$ et alcynoxy(en $C_2$-$C_4$)-alkyle en $C_1$-$C_2$,

$R^4$ est un atome d'hydrogène ou un reste alkyle en $C_1$-$C_4$,

$R^5$ et $R^6$ sont identiques ou différents et représentent un atome d'hydrogène, un reste alkyle en $C_1$-$C_4$ ou des restes (éventuellement substitués par de l'halogène comme en particulier le chlore, le brome ou l'iode) de l'ensemble formé par un reste phényle, benzyle et phényléthyle, et

R représente un reste triiodoalcényle en $C_2$-$C_4$, dibromo-iodoalcényle en $C_2$-$C_4$, dichloro-iodoalcényle en $C_2$-$C_4$, alcynyle en $C_2$-$C_4$ ou iodoalcynyle en $C_2$-$C_4$, les doubles liaisons se trouvant toujours à l'extrémité de la chaîne et, dans le cas d'un reste trihalogénoalcényle, un atome d'iode étant fixé sur le dernier atome de carbone et les deux autres atomes d'halogène sur les deux derniers atomes de carbone de la chaîne, et, dans le cas d'un reste iodoalcynyle, l'atome d'iode étant fixé sur le dernier atome de carbone de la chaîne.

2. Ethers-oxydes de cyclopropylméthyle(ène) substitués selon la formule (I) de la revendication 1, dans lesquels

$X^1$ et $X^2$ sont identiques ou différents et représentent le chlore et/ou le brome,

$R^1$ représente un atome d'hydrogène, un groupe méthyle, éthyle, n-propyle ou n-butyle,

$R^2$ et $R^3$ sont identiques ou différents et représentent un atome d'hydrogène, un reste méthyle, éthyle, n-propyle, n-butyle, 1-pyrazolylméthyle, 1-imidazolylméthyle, 1-(1,2,4-triazolyl)méthyle, méthoxyméthyle, éthoxyméthyle, éthoxyéthyle, méthylthiométhyle, éthylthiométhyle, éthylthioéthyle, (di)méthylaminométhyle, (di)méthylaminoéthyle, (di)éthylaminométhyle, (di)éthylaminoéthyle, propényloxyméthyle, propynyloxyméthyle, iodopropynyloxyméthyle, triiodopropényloxyméthyle, dichloro-iodopropényloxyméthyle, dibromo-iodopropényloxyméthyle, phényle, 2-, 3- ou 4-chlorophényle, 2,4-dichlorophényle, 2-, 3- ou 4-bromophényle, 2,4-dibromophényle, chlorométhyle et bromométhyle,

$R^4$ représente un atome d'hydrogène ou un reste méthyle, éthyle, n- ou isopropyle,

$R^5$ et $R^6$ sont identiques ou différents et représentent un atome d'hydrogène, un reste méthyle, éthyle, n-propyle, isopropyle, 2-, 3- ou 4-chlorophényle, 2,4-dichlorophényle ou 4-bromophényle, et

R représente un reste triiodoéthényle, dichloro-iodoéthényle, dibromo-iodoéthényle, éthynyle, iodoéthynyle, propynyle ou iodopropynyle, et,

dans le cas des restes contenant de l'iode, un atome d'iode est toujours fixé au dernier atome de carbone de la chaîne.

3. Ethers-oxydes de cyclopropylméthyle(ène) substitués selon la formule (I) de la revendication 1, dans lesquels

$X^1$ et $X^2$ sont identiques ou différents et représentent le chlore et/ou le brome,

$R^1$ représente un atome d'hydrogène ou un reste méthyle ou éthyle,

$R^2$ et $R^3$ sont identiques ou différents et représentent un atome d'hydrogène, un reste méthyle, éthyle, chlorométhyle, bromométhyle, 1-propényloxyméthyle, 1-propynyloxyméthyle ou 1-iodopropynyloxyméthyle,

$R^4$ représente un atome d'hydrogène ou un reste méthyle,

$R^5$ et $R^6$ sont identiques ou différents et représentent un atome d'hydrogène ou un reste méthyle, et

R représente un reste triiodoéthényle, dichloroiodoéthényle, dibromo-iodoéthényle, éthynyle ou iodoéthynyle, et, dans le cas des restes contenant de l'iode, un atome d'iode est toujours fixé au dernier atome de carbone de la chaîne.

4. Procédé de préparation d'éthers-oxydes de cyclopropylméthyle(ène) substitués de formule générale (I)

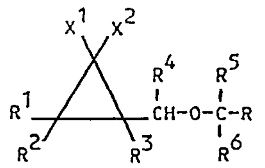

(I)

dans laquelle

$X^1$ et $X^2$ sont identiques ou différents et représentent un atome de fluor, de chlore et/ou de brome,

$R^1$ représente un atome d'hydrogène ou un reste alkyle en $C_1$-$C_6$,

$R^2$ et $R^3$ sont identiques ou différents et représentent un atome d'hydrogène, un reste alkyle en $C_1$-$C_6$, halogénoalkyle en $C_1$-$C_4$, 1-pyrazolylméthyle, 1-imidazolylméthyle, 1-(1,2,4-triazolyl)méthyle, alcoxy(en $C_1$-$C_4$)-alkyle en $C_1$-$C_4$, alkyl(en $C_1$-$C_4$)-thioalkyle en $C_1$-$C_4$, (di)(alkyl en $C_1$-$C_4$)-aminoalkyle en $C_1$-$C_4$ ou des restes (éventuellement substitués par de l'halogène, comme en particulier le chlore, le brome ou l'iode) de l'ensemble formé par un reste phényle, alcénoxy(en $C_2$-$C_4$)-alkyle en $C_1$-$C_2$ et alcynoxy(en $C_2$-$C_4$)-alkyle en $C_1$-$C_2$,

$R^4$ est un atome d'hydrogène ou un reste alkyle en $C_1$-$C_4$,

$R^5$ et $R^6$ sont identiques ou différents et représentent un atome d'hydrogène, un reste alkyle en $C_1$-$C_4$ ou des restes (éventuellement substitués par de l'halogène comme en particulier le chlore, le brome ou l'iode) de l'ensemble formé par un reste phényle, benzyle et phényléthyle, et

R représente un reste triiodoalcényle en $C_2$-$C_4$, dibromo-iodoalcényle en $C_2$-$C_4$, dichloro-iodoalcényle en $C_2$-$C_4$, alcynyle en $C_2$-$C_4$ ou iodoalcynyle en $C_2$-$C_4$, les doubles liaisons se trouvant toujours à l'extrémité de la chaîne et, dans le cas d'un reste trihalogénoalcényle, un atome d'iode étant fixé sur le dernier atome de carbone et les deux autres atomes d'halogène sur les deux derniers atomes de carbone de la chaîne, et, dans le cas d'un reste iodoalcynyle, l'atome d'iode étant fixé sur le dernier atome de carbone de la chaîne, caractérisé en ce que:

a) on fait réagir des halogénures de cyclopropylméthyle(ène) de formule générale (II)

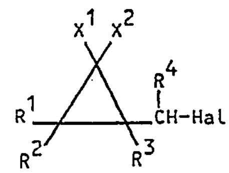

(II)

dans laquelle

$R^1$, $R^2$, $R^3$, $R^4$, $X^1$ et $X^2$ ont le sens indiqué ci-dessus et

Hal représente un halogène comme en particulier le chlore, le brome ou l'iode, avec des alcools de formule générale (III)

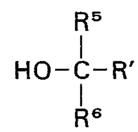

(III)

dans laquelle

$R^5$ et $R^6$ ont le sens indiqué ci-dessus, et

R' représente un reste alcynyle en $C_2$-$C_4$, et la liaison multiple se trouve à l'extrémité de la chaîne, ou leurs sels, éventuellement en présence d'accepteurs d'acides et/ou éventuellement en présence de diluants, ou

b) on fait réagir les nouveaux composés, préparés selon la variante (a) du procédé et répondant à la formule générale (Ia)

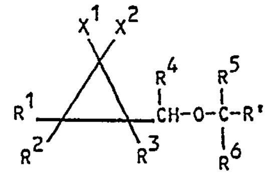

(Ia)

dans laquelle

$R^1$, $R^2$, $R^3$, $R^4$, $R^5$, $R^6$, R', $X^1$ et $X^2$ ont les sens indiqués ci-dessus, avec 0,5 à 6 mol d'iode en présence d'accepteurs d'acides et éventuellement en présence d'un diluant, ou

c) on fait réagir les nouveaux composés, préparés selon le procédé (b) et répondant à la formule générale (Ib)

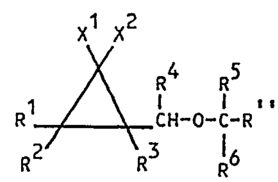

(Ib)

dans laquelle

$R^1$, $R^2$, $R^3$, $R^4$, $R^5$, $R^6$, $X^1$ et $X^2$ ont les sens indiqués ci-dessus, et R'' représente un reste iodoalcynyle en $C_2$-$C_4$, la liaison multiple se trouvant à

l'extrémité de la chaîne et l'atome d'iode étant fixé sur le dernier atome de carbone, avec du chlore, du brome ou de l'iode, en opérant éventuellement en présence d'un diluant.

5. Pesticide, caractérisé en ce qu'il contient au moins un composant de formule (I) selon l'une des revendications 1 ou 4.

6. Pesticide contenant au moins un synergiste et au moins une autre substance active contre les arthropodes, caractérisé en ce qu'il contient au moins un composé de formule (I) selon l'une des revendications 1 ou 4 comme synergiste.

7. Utilisation de composés de formule (I) selon l'une des revendications 1 ou 4 comme synergistes dans la lutte contre les parasites, en particulier des insectes et des arachnides.

8. Procédé pour combattre des parasites, caractérisé en ce qu'on fait agir des composés de formule (I) selon l'une des revendications 1 ou 4 et d'autres substances actives, qui sont actives contre des arthropodes, sur les parasites, de préférence des insectes ou les arachnides ou leur espace vital.

9. Procédé de préparation de pesticides, caractérisé en ce qu'on mélange des composés de formule (I) selon l'une des revendications 1 ou 4 avec d'autres substances actives, qui sont actives contre les arthropodes, et avec des agents d'allongement et/ou des agents tensio-actifs.

## Claims

1. Substituted cyclopropylmethyl(ene) ethers of the Formula (I)

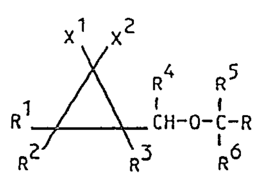

(I)

in which

$X^1$ and $X^2$ are identical or different and represent fluorine, chlorine and/or bromine,

$R^1$ represents hydrogen or $C_1$-$C_6$-alkyl,

$R^2$ and $R^3$ are identical or different and represent hydrogen, $C_1$-$C_6$-alkyl, $C_1$-$C_4$-halogenoalkyl, 1-pyrazolylmethyl, 1-imidazolylmethyl, 1-(1,2,4-triazolyl)methyl, $C_1$-$C_4$-alkoxy-$C_1$-$C_4$-alkyl, $C_1$-$C_4$-alkylthio-$C_1$-$C_4$-alkyl, (di)-$C_1$-$C_4$-alkylamino-$C_1$-$C_4$-alkyl, or radicals from the series comprising phenyl, $C_2$-$C_4$-alkenoxy-$C_1$-$C_2$-alkyl, and $C_2$-$C_4$-alkinoxy-$C_1$-$C_2$-alkyl which are optionally substituted by halogen, such as in particular chlorine, bromine or iodine,

$R^4$ represents hydrogen or $C_1$-$C_4$-alkyl,

$R^5$ and $R^6$ are identical or different and represent hydrogen, $C_1$-$C_4$-alkyl, or radicals from the series comprising phenyl, benzyl and phenethyl which are optionally substituted by halogen, such as in particular chlorine, bromine or iodine, and R represents triiodo-$C_2$-$C_4$-alkenyl, dibromoiodo-$C_2$-$C_4$-alkenyl, dichloroiodo-$C_2$-$C_4$-alkenyl, $C_2$-$C_4$-alkinyl or iodo-$C_2$-$C_4$-alkinyl, the multiple bonds being on the end of the chain in each case and, in the case of trihalogenoalkenyl, the iodine atom being bonded to the last carbon atom and the two other halogen atoms being bonded to the last two carbon atoms and, in the case of iodoalkinyl, the iodine atom being bonded to the last carbon atom of the chain.

2. Substituted cyclopropylmethyl(ene) ethers of the Formula (I) according to Claim 1, in which $X^1$ and $X^2$ are identical or different and represent chlorine and/or bromine,

$R^1$ represents hydrogen, methyl, ethyl, n-propyl or n-butyl,

$R^2$ and $R^3$ are identical or different and represent hydrogen, methyl, ethyl, n-propyl, n-butyl, 1-pyrazolylmethyl, 1-imidazolylmethyl, 1-(1,2,4-triazolyl)methyl, methoxymethyl, ethoxymethyl, ethoxyethyl, methylthiomethyl, ethylthiomethyl, ethylthioethyl, (di)methylaminomethyl, (di)methylaminoethyl, (di)ethylaminomethyl, (di)ethylaminoethyl, propenyloxymethyl, propinyloxymethyl, iodopropinyloxymethyl, triiodopropenyloxymethyl, dichloroiodopropenyloxymethyl, dibromoiodopropenyloxymethyl, phenyl, 2-, 3-, 4-chlorophenyl, 2,4-dichlorophenyl, 2-, 3-, 4-bromophenyl, 2,4-dibromophenyl, chloromethyl or bromomethyl,

$R^4$ represents hydrogen, methyl, ethyl or n- or i-propyl,

$R^5$ and $R^6$ are identical or different and represent hydrogen, methyl, ethyl, n-propyl, i-propyl, 2-, 3-, 4-chlorophenyl, 2,4-dichlorophenyl or 4-bromophenyl, and

R represents triiodoethenyl, dichloroiodoethenyl, dibromoiodoethenyl, ethinyl, iodoethinyl, propinyl or iodopropinyl, one iodine atom in each of the iodine-containing radicals being bonded to the last carbon atom of the chain.

3. Substituted cyclopropylmethyl(ene) ethers of the Formula (I) according to Claim 1, in which

$X^1$ and $X^2$ are identical or different and represent chlorine and/or bromine,

$R^1$ represents hydrogen, methyl or ethyl,

$R^2$ and $R^3$ are identical or different and represent hydrogen, methyl, ethyl, chloromethyl, bromomethyl, 1-propenyloxymethyl, 1-propinyloxymethyl or 1-iodopropinyloxymethyl,

$R^4$ represents hydrogen or methyl,

$R^5$ and $R^6$ are identical or different and represent hydrogen or methyl, and

$R^7$ represents triiodoethenyl, dichloroiodoethenyl, dibromoiodoethenyl, ethinyl or iodoethinyl, one iodine atom in each of the iodine-containing radicals being bonded to the last carbon atom of the chain.

4. Process for the preparation of substituted cyclopropylmethyl(ene) ethers of the general Formula (I)

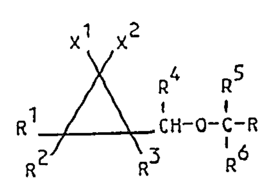

(I)

in which

$X^1$ and $X^2$ are identical or different and represent fluorine, chlorine and/or bromine,

$R^1$ represents hydrogen or $C_1$-$C_6$-alkyl,

$R^2$ and $R^3$ are identical or different and represent hydrogen, $C_1$-$C_6$-alkyl, $C_1$-$C_4$-halogenoalkyl, 1-pyrazolylmethyl, 1-imidazolylmethyl, 1-(1,2,4-triazolyl)methyl, $C_1$-$C_4$-alkoxy-$C_1$-$C_4$-alkyl, $C_1$-$C_4$-alkylthio-$C_1$-$C_4$-alkyl, (di)-$C_1$-$C_4$-alkylamino-$C_1$-$C_4$-alkyl, or radicals from the series comprising phenyl, $C_2$-$C_4$-alkenoxy-$C_1$-$C_2$-alkyl and $C_2$-$C_4$-alkinoxy-$C_1$-$C_2$-alkyl which are optionally substituted by halogen, such as, in particular, chlorine, bromine or iodine,

$R^4$ represents hydrogen or $C_1$-$C_4$-alkyl,

$R^5$ and $R^6$ are identical or different and represent hydrogen, $C_1$-$C_4$-alkyl, or radicals from the series comprising phenyl, benzyl and phenethyl which are optionally substituted by halogen, such as in particular chlorine, bromine or iodine, and

R represents triiodo-$C_2$-$C_4$-alkenyl, dibromoiodo-$C_2$-$C_4$-alkenyl, dichloroiodo-$C_2$-$C_4$-alkenyl, $C_2$-$C_4$-alkinyl or iodo-$C_2$-$C_4$-alkinyl, the multiple bonds being on the end of the chain in each case and, in the case of trihalogenoalkenyl, one iodine atom being bonded to the last carbon atom and the two other halogen atoms being bonded to the last two carbon atoms and, in the case of iodoalkinyl, the iodine atom being bonded to the last carbon atom of the chain, characterised in that

(*a*) cyclopropylmethyl(ene) halides of the general Formula (II)

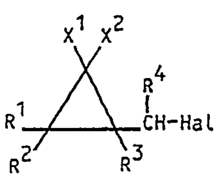

(II)

in which

$R^1$, $R^2$, $R^3$, $R^4$, $X^1$ and $X^2$ have the above-mentioned meaning, and

Hal represents halogen, such as in particular chlorine, bromine or iodine, are reacted with alcohols of the general Formula (III)

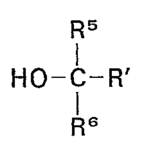

(III)

in which

$R^5$ and $R^6$ have the above-mentioned meaning, and

R′ represents $C_2$-$C_4$-alkinyl, the multiple bond being at the end of the chain, or salts thereof, if appropriate in the presence of acid acceptors and/or if appropriate in the presence of diluents, or

(*b*) the new compounds, prepared according to process variant (*a*), of the general Formula (Ia)

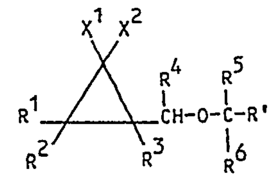

(Ia)

in which

$R^1$, $R^2$, $R^3$, $R^4$, $R^5$, $R^6$, R′, $X^1$ and $X^2$ have the above-mentioned meanings, are reacted with 0.5 to 6 mol of iodine in the presence of acid acceptors and, if appropriate, in the presence of a diluent, or

(*c*) the new compounds, prepared according to process (*b*), of the general Formula (Ib)

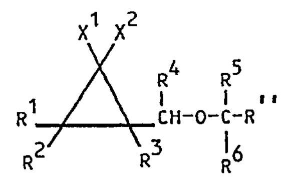

(Ib)

in which

$R^1$, $R^2$, $R^3$, $R^4$, $R^5$, $R^6$, $X^1$ and $X^2$ have the above-mentioned meanings and

R″ represents iodo-$C_2$-$C_4$-alkinyl, the multiple bond being at the end of the chain and the iodine atome being bonded to the last carbon atom, are reacted with chlorine, bromine or iodine, if appropriate in the presence of a diluent.

5. Agents for combating pests, characterised in that they contain at least one compound of the Formula (I) according to Claim 1 or 4.

6. Agents for combating pests, containing at least one synergist and at least one other substance active against arthropods, characterised in that they contain at least one compound of the Formula (I) according to Claim 1 or 4 as the synergist.

7. Use of compounds of the Formula (I) according to Claim 1 or 4 as synergists in combating pests, in particular insects and arachnids.

8. Method of combating pests, characterised in that compounds of the Formula (I) according to Claim 1 or 4 and other active compounds which are active against arthropods are allowed to act on the pests, preferably insects or arachnids, or their environment.

9. Process for the preparation of agents for combating pests, characterised in that compounds of the Formula I according to Claim 1 or 4 are mixed with other active compounds which are active against arthropods, and with extenders and/or surface-active agents.